# EUROPEAN PATENT SPECIFICATION

(11) **EP 4 331 482 B1**
(45) Date of publication and mention of the grant of the patent: **02.09.2026**
(21) Application number: 22846104.2
(22) Date of filing: 07.07.2022
(51) Int. Cl.: A61B 5/11, A61B 5/024, A61B 5/00, A63B 24/00

(54) **ELECTRONIC DEVICES COMPRISING SENSORS AND METHOD FOR OPERATING SAME**
ELEKTRONISCHE VORRICHTUNGEN MIT SENSOREN UND VERFAHREN ZUM BETRIEB DAVON
DISPOSITIFS ÉLECTRONIQUES COMPRENANT DES CAPTEURS ET PROCÉDÉ DE FONCTIONNEMENT ASSOCIÉ

(30) Priority: 19.07.2021 KR 20210094061
(43) Date of publication of application: 06.03.2024
(73) Proprietor: Samsung Electronics Co., Ltd., Suwon-si, Gyeonggi-do 16677 (KR)
(72) Inventor: KIM, Jinik, Suwon-si Gyeonggi-do 16677 (KR); PARK, Namjoon, Suwon-si Gyeonggi-do 16677 (KR)
(74) Representative: HGF
(86) International application number: PCT/KR2022/009825
(87) International publication number: WO 2023/003232

(56) References cited:
- EP-A1- 3 190 475
- WO-A1-2015/131065
- WO-A1-2021/016536
- JP-A- 2004 184 351
- JP-A- 2019 005 503
- KR-A- 20160 035 394
- KR-A- 20170 118 971
- KR-B1- 101 715 369
- KR-B1- 101 715 369
- KR-B1- 102 254 164
- US-A1- 2016 081 625
- US-A1- 2016 256 058
- GRUSKAY JORDAN A. ET AL: "Pediatric and adolescent ankle instability: diagnosis and treatment options", CURRENT OPINION IN PEDIATRICS., vol. 31, no. 1, 1 February 2019 (2019-02-01), US, pages 69 - 78, XP093191158, ISSN: 1040-8703, Retrieved from the Internet <URL:https://dx.doi.org/10.1097/MOP.0000000000000720> DOI: 10.1097/MOP.0000000000000720

## Description

### BACKGROUND

### 1. Technical Field

The disclosure relates to multi-device tracking of user activity, and more particularly, to using multiple electronic devices to track a user's exercise activity.

### 2. Description of Related Art

Recent times have seen increase in the use of wearable devices, such as smart watches and wireless earphones, which may interoperate with other electronic devices, such as mobile devices and smartphones. A wearable device may be worn on a part of a user's body, such as their wrist or ear, and may be used to measure biometric information for use with, for example, exercise and health analytics. Accordingly, it would be desirable to develop technologies and services for providing users with useful information via interoperation between wearable devices and mobile devices.

US2016/081625 A1 discloses a method and apparatus for processing sensor data.

EP3190475 A1 discloses an information terminal, motion capture system, and motion capture method.

US2016/256058 A1 discloses a statistical heart rate monitoring for estimating calorie expenditure.

WO2015/131065 A1 discloses a method and apparatus for generating assessments using physical activity and biometric parameters.

WO2021/016536 A1 discloses systems and methods for operating an implantable medical device based upon sensed posture information.

### SUMMARY

In certain example embodiments of the present disclosure, exercise-related information may be provided via interoperation of wearable devices and electronic devices, such as an exercise starting timepoint, posture and exercise-type, based on information detected via a wearable device worn on the body of a user.

However, the technical aspects are not limited to the aforementioned aspects, and other technical aspects may be present.

In certain embodiments of the disclosure, a method of operating an electronic device interoperating with a plurality of external electronic devices is disclosed, including receiving, via a communication circuit, first sensor data including a heart rate, second sensor data indicating a posture of a first external electronic device from among the plurality of external electronic devices, and third sensor data indicating relative position between the plurality of external electronic devices; determining, via at least one processor, an exercise starting timepoint based on the first sensor data; estimating an exercise posture of a user based on the second sensor data; estimating a pattern of change in distance between each of the plurality of external electronic devices, indicated by changes in the relative position between each the plurality of external electronic devices over a time period after the determined exercise starting timepoint, and based on the third sensor data; and generating exercise information based on the estimated exercise posture and the estimated pattern of change in distance between the plurality of external electronic devices.

According to certain embodiments of the disclosure, an electronic device is disclosed, including: a communication circuit configured to communicate with a plurality of external electronic devices that are wearable on a body of a user; at least one processor configured to: determine an exercise starting timepoint based on first sensor data, estimate an exercise posture of the user based on second sensor data, estimate a pattern of change in distance between each of the plurality of external electronic devices, indicated by changes in a relative position between each the plurality of external electronic devices over a time period after the determined exercise starting timepoint, and based on third sensor data, and generate exercise information based on the estimated exercise posture and the estimated pattern of change in distance between the plurality of external electronic devices.

According to certain embodiments of the disclosure, a wearable device interoperating with at least one external electronic device is disclosed, including: a sensor module including at least one sensor; and an output module; at least one processor configured to: determine an exercise starting timepoint of a user wearing the wearable device based on first sensor data detected via the sensor module, including a heart rate of the user, estimate an exercise posture of the user based on second sensor data indicating a posture of the wearable device as detected by the sensor module, estimate a pattern of change in distance with the external electronic device based on the exercise starting timepoint, and on third sensor data detected by the sensor module monitoring distance with the external electronic device over at least a time period after the exercise starting timepoint, generate exercise information based at least on the estimated exercise posture, and the estimated pattern of change in the distance with the external electronic device, and output the generated exercise information via the output module.

According to certain example embodiments of the present disclosure, postures of a user may be subdivided and classified using an electronic device and at least one external electronic device, and detailed exercise information for each exercise category may be provided to the user based on sensor data of external electronic devices attached to different parts of the user.

According to certain example embodiments of the present disclosure, an actual exercise starting timepoint of a user may be accurately determined using a change pattern indicated in of biometric information received through a biometric sensor of the electronic device. Detailed exercises for each exercise category may be automatically classified using an inertial sensor and a distance sensor. In addition, subdivided and detailed exercise information may be provided to the user after an exercise is completed. The invention is defined in the independent claims.

### BRIEF DESCRIPTION OF THE DRAWINGS

The above and other aspects, features, and advantages of certain embodiments of the present disclosure will be more apparent from the following detailed description, taken in conjunction with the accompanying drawings, in which:
FIG. 1 is a block diagram illustrating an electronic device in a network environment according to certain example embodiments;
FIGS. 2A and 2B are perspective views of an electronic device according to an example embodiment;
FIG. 3 is an exploded perspective view of an electronic device according to an example embodiment;
FIG. 4 is a block diagram of an electronic device interoperating with an external electronic device according to certain example embodiments;
FIG. 5 is a flowchart illustrating a method of operating an electronic device according to certain example embodiments;
FIGS. 6A and 6B are diagrams illustrating photoplethysmography (PPG) waveforms acquired from a first user and a second user, respectively, according to certain example embodiments;
FIG. 7A is a diagram illustrating a method of correcting a posture value based on a body coordinate system to a posture value based on a North East Up (NEU) coordinate system, according to certain example embodiments;
FIG. 7B is a diagram illustrating a method of correcting posture values related to roll and pitch to values parallel to a surface of the earth perpendicular to gravity, according to certain example embodiments;
FIGS. 8A to 8C are diagrams illustrating posture values of a first external electronic device corresponding to an exercise posture of a user, according to certain example embodiments;
FIG. 9 is a diagram illustrating a method of filtering a pattern of signals included in sensor data, according to certain example embodiments;
FIG. 10 is a diagram illustrating a method of generating information on an exercise part according to certain example embodiments;
FIGS. 11A and 11B are diagrams illustrating an interface output from a display of an electronic device according to certain example embodiments;
FIGS. 12A and 12B are diagrams illustrating an interface output from a display of an external electronic device according to certain example embodiments;
FIG. 13 is a block diagram of a wearable device interoperating with an external electronic device according to certain example embodiments; and
FIG. 14 is a flowchart illustrating a method of operating a wearable device according to certain example embodiments.

### DETAILED DESCRIPTION

Hereinafter, example embodiments will be described in detail with reference to the accompanying drawings. When describing the example embodiments with reference to the accompanying drawings, like reference numerals refer to like elements and a repeated description related thereto will be omitted.

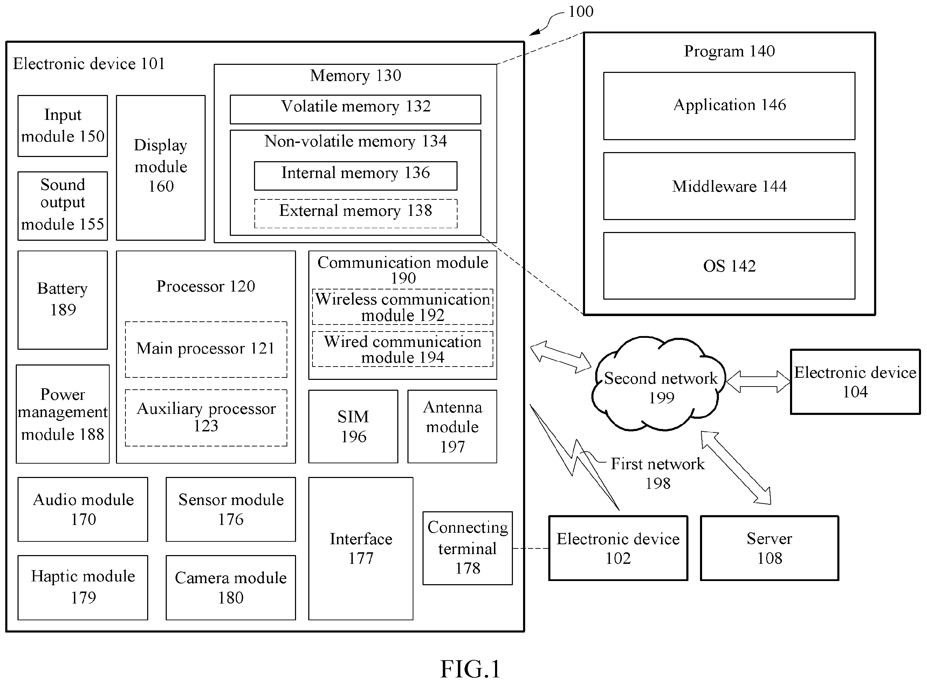

FIG. 1 is a block diagram illustrating an electronic device 101 in a network environment 100 according to certain example embodiments. Referring to FIG. 1, the electronic device 101 in the network environment 100 may communicate with an electronic device 102 via a first network 198 (e.g., a short-range wireless communication network), or communicate with an electronic device 104 or a server 108 via a second network 199 (e.g., a long-range wireless communication network). According to an example embodiment, the electronic device 101 may communicate with the electronic device 104 via the server 108. According to an example embodiment, the electronic device 101 may include a processor 120, a memory 130, an input module 150, a sound output module 155, a display module 160, an audio module 170, and a sensor module 176, an interface 177, a connecting terminal 178, a haptic module 179, a camera module 180, a power management module 188, a battery 189, a communication module 190, a subscriber identification module (SIM) 196, or an antenna module 197. In some example embodiments, at least one of the components (e.g., the connecting terminal 178) may be omitted from the electronic device 101, or one or more other components may be added in the electronic device 101. In some example embodiments, some of the components (e.g., the sensor module 176, the camera module 180, or the antenna module 197) may be integrated as a single component (e.g., the display module 160).

The processor 120 may execute, for example, software (e.g., a program 140) to control at least one other component (e.g., a hardware or software component) of the electronic device 101 connected to the processor 120, and may perform various data processing or computation. According to an example embodiment, as at least a part of data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an example embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with the main processor 121. For example, when the electronic device 101 includes the main processor 121 and the auxiliary processor 123, the auxiliary processor 123 may be adapted to consume less power than the main processor 121 or to be specific to a specified function. The auxiliary processor 123 may be implemented separately from the main processor 121 or as a part of the main processor 121.

The auxiliary processor 123 may control at least some of functions or states related to at least one (e.g., the display module 160, the sensor module 176, or the communication module 190) of the components of the electronic device 101, instead of the main processor 121 while the main processor 121 is in an inactive (e.g., sleep) state or along with the main processor 121 while the main processor 121 is an active state (e.g., executing an application). According to an example embodiment, the auxiliary processor 123 (e.g., an ISP or a CP) may be implemented as a portion of another component (e.g., the camera module 180 or the communication module 190) that is functionally related to the auxiliary processor 123. According to an example embodiment, the auxiliary processor 123 (e.g., an NPU) may include a hardware structure specified for artificial intelligence model processing. An artificial intelligence model may be generated by machine learning. Such learning may be performed by, for example, the electronic device 101 in which artificial intelligence is performed, or performed via a separate server (e.g., the server 108). Learning algorithms may include, but are not limited to, for example, supervised learning, unsupervised learning, semi-supervised learning, or reinforcement learning. The AI model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), and a bidirectional recurrent deep neural network (BRDNN), a deep Q-network, or a combination of two or more thereof, but is not limited thereto. The AI model may additionally or alternatively include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134. The non-volatile memory 134 may itself include internal memory 136 and external memory 138.

The program 140 may be stored as software in the memory 130, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output a sound signal to the outside of the electronic device 101. The sound output module 155 may include, for example, a speaker or a receiver. The speaker may be used for general purposes, such as playing multimedia or playing record. The receiver may be used to receive an incoming call. According to an example embodiment, the receiver may be implemented separately from the speaker or as a part of the speaker.

The display module 160 may visually provide information to the outside (e.g., a user) of the electronic device 101. The display module 160 may include, for example, a control circuit for controlling a display, a hologram device, or a projector and control circuitry to control a corresponding one of the display, the hologram device, and the projector. According to an example embodiment, the display module 160 may include a touch sensor adapted to detect a touch, or a pressure sensor adapted to measure the intensity of force incurred by the touch.

The audio module 170 may convert a sound into an electric signal or vice versa. According to an example embodiment, the audio module 170 may obtain the sound via the input module 150 or output the sound via the sound output module 155 or an external electronic device (e.g., the electronic device 102 such as a speaker or a headphone) directly or wirelessly connected to the electronic device 101.

The sensor module 176 may detect an operational state (e.g., power or temperature) of the electronic device 101 or an environmental state (e.g., a state of a user) external to the electronic device 101, and generate an electric signal or data value corresponding to the detected state. According to an example embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an example embodiment, the interface 177 may include, for example, a high-definition multimedia interface (HDMI), a universal serial bus (USB) interface, a secure digital (SD) card interface, or an audio interface.

The connecting terminal 178 may include a connector via which the electronic device 101 may be physically connected to an external electronic device (e.g., the electronic device 102). According to an example embodiment, the connecting terminal 178 may include, for example, an HDMI connector, a USB connector, an SD card connector, or an audio connector (e.g., a headphone connector).

The haptic module 179 may convert an electric signal into a mechanical stimulus (e.g., a vibration or a movement) or an electrical stimulus which may be recognized by a user via his or her tactile sensation or kinesthetic sensation. According to an example embodiment, the haptic module 179 may include, for example, a motor, a piezoelectric element, or an electric stimulator.

The camera module 180 may capture a still image and moving images. According to an example embodiment, the camera module 180 may include one or more lenses, image sensors, image signal processors, or flashes.

The power management module 188 may manage power supplied to the electronic device 101. According to an example embodiment, the power management module 188 may be implemented as, for example, at least a part of a power management integrated circuit (PMIC).

The battery 189 may supply power to at least one component of the electronic device 101. According to an example embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently of the processor 120 (e.g., an AP) and that support a direct (e.g., wired) communication or a wireless communication. According to an example embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module, or a power line communication (PLC) module). A corresponding one of these communication modules may communicate with the external electronic device 104 via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., a LAN or a wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the SIM 196.

The wireless communication module 192 may support a 5G network after a 4G network, and a next-generation communication technology, e.g., a new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., a mmWave band) to achieve, e.g., a high data transmission rate. The wireless communication module 192 may support various technologies for securing performance on a high-frequency band, such as, e.g., beamforming, massive multiple-input and multiple-output (MIMO), full dimensional MIMO (FD-MIMO), an array antenna, analog beam-forming, or a large scale antenna. The wireless communication module 192 may support various requirements specified in the electronic device 101, an external electronic device (e.g., the electronic device 104), or a network system (e.g., the second network 199). According to an example embodiment, the wireless communication module 192 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., the external electronic device) of the electronic device 101. According to an example embodiment, the antenna module 197 may include an antenna including a radiating element including a conductive material or a conductive pattern formed in or on a substrate (e.g., a printed circuit board (PCB)). According to an example embodiment, the antenna module 197 may include a plurality of antennas (e.g., array antennas). In such a case, at least one antenna appropriate for a communication scheme used in a communication network, such as the first network 198 or the second network 199, may be selected by, for example, the communication module 190 from the plurality of antennas. The signal or the power may be transmitted or received between the communication module 190 and the external electronic device via the at least one selected antenna. According to an example embodiment, another component (e.g., a radio frequency integrated circuit (RFIC)) other than the radiating element may be additionally formed as a part of the antenna module 197.

According to certain example embodiments, the antenna module 197 may form a mmWave antenna module. According to an example embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an example embodiment, commands or data may be transmitted or received between the electronic device 101 and the external electronic device 104 via the server 108 coupled with the second network 199. Each of the external electronic devices 102 or 104 may be a device of the same type as or a different type from the electronic device 101. According to an example embodiment, all or some of operations to be executed by the electronic device 101 may be executed at one or more external electronic devices (e.g., the external devices 102 and 104, and the server 108). For example, if the electronic device 101 needs to perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and may transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an example embodiment, the external electronic device 104 may include an Internet-of-things (IoT) device. The server 108 may be an intelligent server using machine learning and/or a neural network. According to an example embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

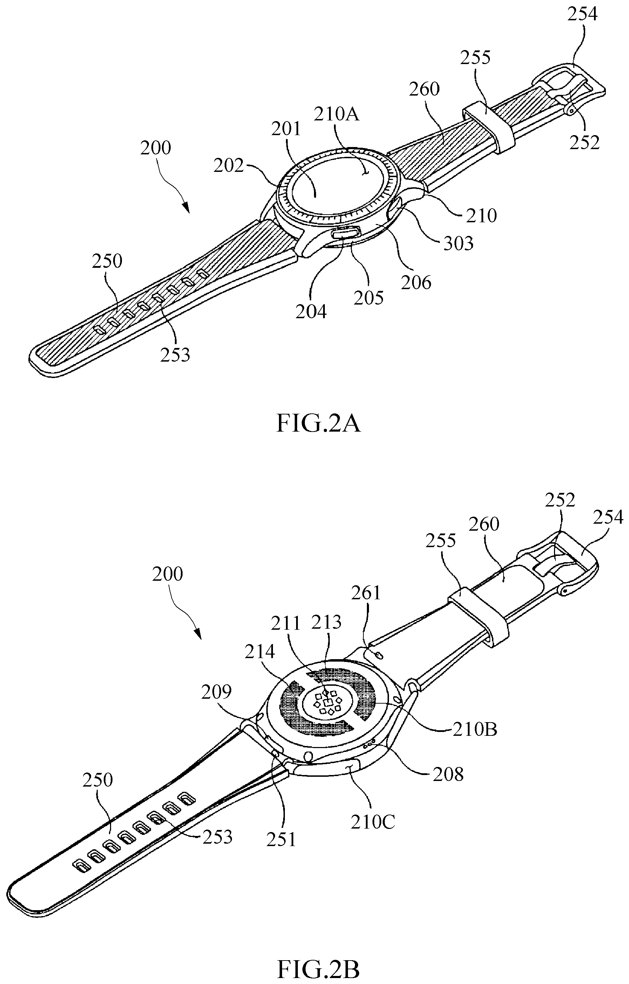

Referring to FIGS. 2A and 2B, according to an example embodiment, an electronic device 200 (e.g., the electronic device 101 of FIG. 1) may include a housing 210 including a first surface (or front surface) 210A, a second surface (or rear surface) 210B, and a side surface 210C surrounding a space between the first surface 210A and the second surface 210B, and fastening members 250 and 260 connected to at least a portion of the housing 210 and configured to detachably attach the electronic device 200 to a body part (e.g, a wrist, or an ankle) of a user. In an example embodiment (not shown), the housing may also refer to a structure which forms a portion of the first face 210A, the second face 210B, and the side face 210C of FIG. 2A. According to an example embodiment, the first surface 210A may be formed by a front plate 201 (e.g., a glass plate or a polymer plate including various coating layers) of which at least a portion is substantially transparent. The second surface 210B may be formed by a rear plate 207 that is substantially opaque. The rear plate 207 may be formed of, for example, coated or colored glass, ceramic, polymer, metal (e.g., aluminum, stainless steel (STS), or magnesium), or a combination of at least two thereof. The side surface 210C may be coupled to the front plate 201 and the rear plate 207 and may be formed by a side bezel structure (or a "side member") 206 including a metal and/or a polymer. In an example embodiment, the rear plate 207 and the side bezel structure 206 may be integrally formed and may include the same material (e.g., a metal material such as aluminum). The fastening members 250 and 260 may be formed of various materials and may have various shapes. For example, the fastening members 250 and 260 may be formed of woven fabric, leather, rubber, urethane, metal, ceramic, or a combination of at least two of the aforementioned materials and may be implemented in an integrated form or with a plurality of unit links that are movable relative to each other.

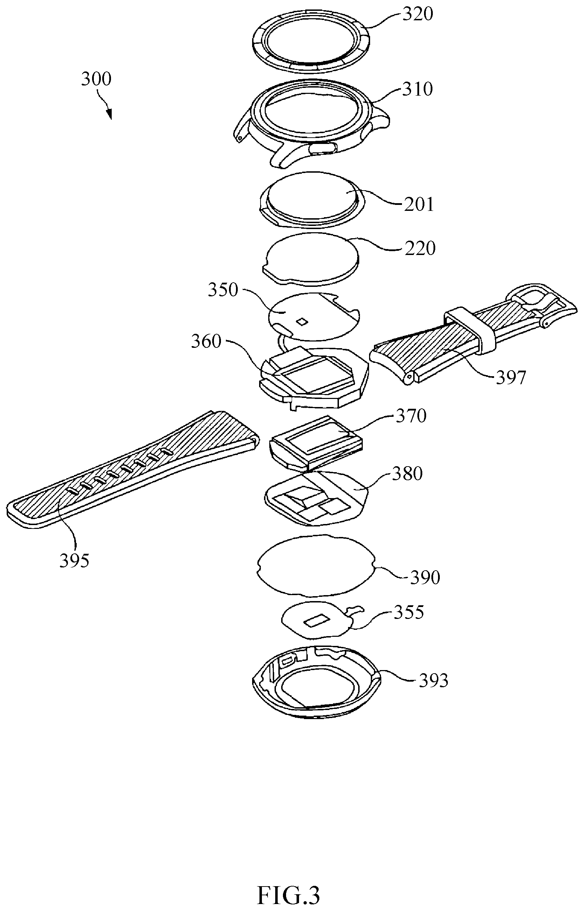

According to an example embodiment, the electronic device 200 may include at least one of a display 220 (refer to FIG. 3), audio modules(e.g., a microphone hole 205 or a speaker hole 208), a sensor module 211, key input devices 202, 203, and 204, and a connector hole 209. In an example embodiment, the electronic device 200 may not include at least one (e.g., the key input devices 202, 203, and 204, the connector hole 209, or the sensor module 211) of the components, or additionally include other components.

The display 220 may be exposed through, for example, some portions of the front plate 201. The display 220 may have a shape corresponding to a shape of the front plate 201, and may have various shapes such as a circle, an oval, or a polygon. The display 220 may be coupled to or disposed adjacent to a touch sensing circuit, a pressure sensor capable of measuring an intensity (or pressure) of a touch, and/or a fingerprint sensor.

The audio modules may include a microphone hole 205 and a speaker hole 208. A microphone for acquiring an external sound may be disposed in the microphone hole 205. In some example embodiments, a plurality of microphones may be disposed to detect a direction of a sound. The speaker hole 208 may be used as an external speaker and a call receiver for calls. In an example embodiment, the speaker hole 208 and the microphone hole 205 may be implemented as a single hole, or a speaker (e.g., a piezo speaker) may be included without the speaker hole 208.

The sensor module 211 may generate an electrical signal or a data value corresponding to an internal operating state of the electronic device 200 or an external environmental state. The sensor module 211 may include, for example, a biometric sensor module 211 (e.g., a heart rate monitor (HRM) sensor) disposed on the second surface 210B of the housing 210. The electronic device 200 may further include at least one of sensor modules (not shown), for example, a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illuminance sensor.

The sensor module 211 may include electrode areas 213 and 214 that form a portion of the surface of the electronic device 200 and a biosignal detection circuit (not shown) electrically connected to the electrode areas 213 and 214. For example, the electrode areas 213 and 214 may include a first electrode area 213 and a second electrode area 214 disposed on the second surface 210B of the housing 210. The sensor module 211 may be configured such that the electrode areas 213 and 214 obtain electrical signals from a part of the user's body and the biosignal detection circuit detects biometric information of the user based on the electrical signals.

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key 202 may have a shape corresponding to the shape of the front plate 201. In an example embodiment, the electronic device 200 may not include some or all of the above-described key input devices 202, 203, 204, and the key input devices 202, 203, and 204 that are not included may be implemented in other forms such as soft keys on the display 220. The connector hole 209 may accommodate a connector (e.g., a universal serial bus (USB) connector) for transmitting and receiving power and/or data to and from an external electronic device and may include another connector hole (not shown) that accommodates a connector for transmitting and receiving an audio signal to and from an external electronic device. The electronic device 200 may further include, for example, a connector cover (not shown) that covers at least a portion of the connector hole 209 and blocks infiltration of external foreign materials into the connector hole 209.

The fastening members 250 and 260 may be detachably fastened to at least a partial area of the housing 210 using locking members 251 and 261. The fastening members 250 and 260 may include one or more of a fixing member 252, a fixing member fastening hole 253, a band guide member 254, and a band fixing ring 255.

The fixing member 252 may be configured to fix the housing 210 and the fastening members 250 and 260 to a part (e.g., a wrist, an ankle, etc.) of the user's body. The fixing member fastening hole 253 may correspond to the fixing member 252 to fix the housing 210 and the fastening members 250 and 260 to the part of the user's body. The band guide member 254 may be configured to limit a range of a movement of the fixing member 252 when the fixing member 252 is fastened to the fixing member fastening hole 253, so that the fastening members 250 and 260 may be fastened to the part of the user's body in a state of being brought into close contact with the part of the user's body. The band fixing ring 255 may limit a range of a movement of the fastening members 250 and 260 in a state in which the fixing member 252 and the fixing member fastening hole 253 are fastened with each other.

Referring to FIG. 3, an electronic device 300 (e.g., the electronic device 101 of FIG. 1 or the electronic device 200 of FIGS. 2A and 2B) may include a side bezel structure 310, a wheel key 320, a front plate 201, a display 220, a first antenna 350, a second antenna 355, a support member 360 (e.g., a bracket), a battery 370, a PCB 380, a sealing member 390, a rear plate 393, and fastening members 395 and 397. At least one of the components of the electronic device 300 may be the same as or similar to at least one of the components of the electronic device 101 of FIG. 1 or the electronic device 200 of FIGS. 2A and 2B, and accordingly a repeated description thereof will be omitted hereinafter. The support member 360 may be disposed inside the electronic device 300 and may be connected to the side bezel structure 310, or may be integrally formed with the side bezel structure 310. The support member 360 may be formed of, for example, a metal material and/or a non-metal material (e.g., polymer). The display 220 may be coupled to one surface of the support member 360, and the PCB 380 may be coupled to the other surface of the support member 360. The PCB 380 may be provided with a processor, a memory, and/or an interface mounted thereon. The processor may include, for example, one or more of a CPU, a GPU, an AP, a sensor processor, or a communication processor.

The memory may include, for example, a volatile memory or a non-volatile memory. The interface may include, for example, an HDMI, a USB interface, an SD card interface, or an audio interface. For example, the interface may electrically or physically connect the electronic device 300 to an external electronic device, and may include a USB connector, an SD card/multimedia card (MMC) connector, or an audio connector.

The battery 370, which is a device for supplying power to at least one component of the electronic device 300, may include, for example, a non-rechargeable primary battery, a rechargeable secondary battery, or a fuel cell. For example, at least a portion of the battery 370 may be disposed on substantially the same plane as the PCB 380. The battery 370 may be disposed integrally inside the electronic device 300, or disposed detachably from the electronic device 300.

The first antenna 350 may be disposed between the display 220 and the support member 360. The first antenna 350 may include, for example, a near-field communication (NFC) antenna, a wireless charging antenna, and/or a magnetic secure transmission (MST) antenna. For example, the first antenna 350 may perform short-range communication with an external device, wirelessly transmit and receive power used for charging, or transmit a magnetism-based signal including a short-range communication signal or payment data. In an example embodiment, an antenna structure may be formed by a portion of the side bezel structure 310 and/or the support member 360, or a combination thereof.

The second antenna 355 may be disposed between the PCB 380 and the rear plate 393. The second antenna 355 may include, for example, an NFC antenna, a wireless charging antenna, and/or an MST antenna. For example, the second antenna 355 may perform short-range communication with an external device, wirelessly transmit and receive power used for charging, or transmit a magnetism-based signal including a short-range communication signal or payment data. In an example embodiment, an antenna structure may be formed by a portion of the side bezel structure 310 and/or the rear plate 393, or a combination thereof.

The sealing member 390 may be disposed between the side bezel structure 310 and the rear plate 393. The sealing member 390 may be configured to prevent moisture and foreign materials from being introduced into a space surrounded by the side bezel structure 310 and the side plate 393 from the outside.

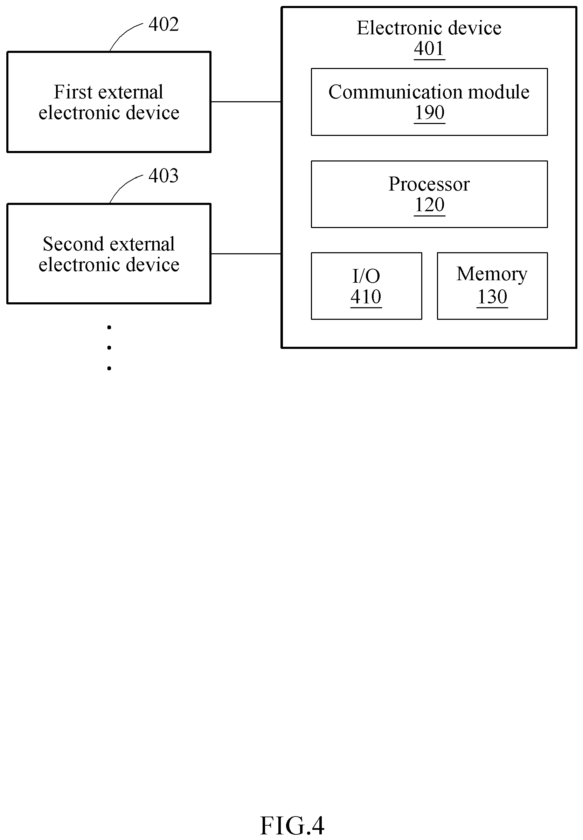

FIG. 4 is a block diagram of an electronic device interoperating with an external electronic device according to certain example embodiments.

Referring to FIG. 4, an electronic device 401 (e.g., the electronic device 101 of FIG. 1) according to an example embodiment may include a communication module 190 (e.g., the communication module 190 of FIG. 1), a processor 120 (e.g., the processor 120 of FIG. 1), an input/output (I/O) module 410 (e.g., the input module 150, the sound output module 155, and/or the display module 160 of FIG. 1), and a memory 130 (e.g., the memory 130 of FIG. 1), and may interoperate with a plurality of external electronic devices 402 and 403. The electronic device 401 may include, for example, a mobile terminal, a tablet, or a personal computer (PC). In addition to the configuration shown in FIG. 4, the electronic device 401 may further include the configuration of the electronic device 101 described above with reference to FIG. 1, such as a sensor module (e.g., the sensor module 176 of FIG. 1).

The external electronic devices 402 and 403 according to an example embodiment may be electronic devices wearable on a body of a user of an electronic device, and may include, for example, a wearable device such as a patch or a smart watch, and a hearable device such as an earphone. The external electronic devices 402 and 403 may each include a processor, a communication module, and at least one sensor. The external electronic devices 402 and 403 may communicate with the electronic device 401 that interoperates with the external electronic devices 402 and 403, using the communication module, and may transmit, for example, data sensed by the sensor to the electronic device 401.

The sensor may be an element or device that measures a physical quantity (e.g., a temperature, light, color, a pressure, magnetism, a speed, or an acceleration) of an object and replaces the physical quantity with a signal (e.g., an electric signal) that may be processed by a machine. The sensor may include, for example, a biometric sensor, a gyro sensor, an acceleration sensor, a geomagnetic sensor, a distance sensor, a height sensor, and a pressure sensor.

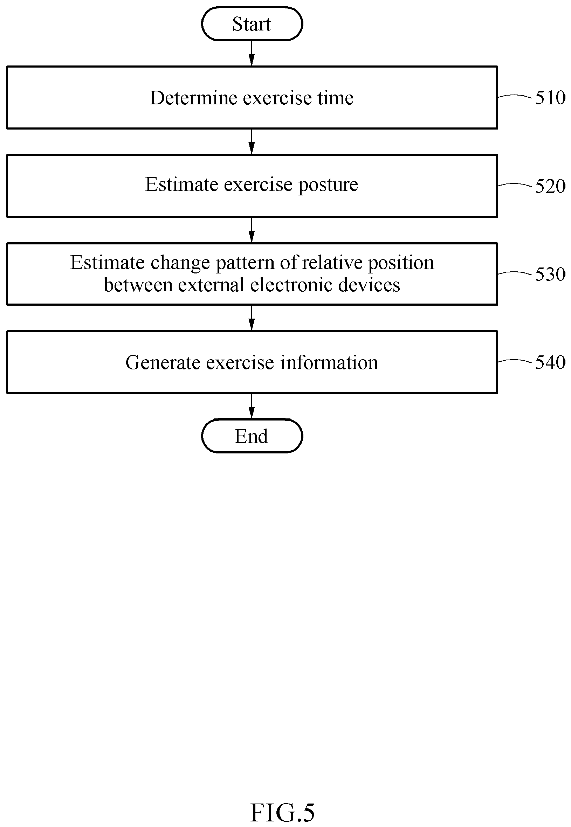

FIG. 5 is a flowchart illustrating a method of operating an electronic device according to certain example embodiments.

Referring to FIG. 5, a method of operating an electronic device (e.g., the electronic device 101 of FIG. 1 or the electronic device 401 of FIG. 4) according to an example embodiment may include operation 510 of determining an exercise starting timepoint, operation 520 of estimating an exercise posture, operation 530 of estimating a change pattern of a relative position between external electronic devices, and operation 540 of generating exercise information. The method of FIG. 5 may be performed by, for example, a processor (e.g., the processor 120 of FIG. 1 or the processor 120 of FIG. 4) of the electronic device.

More specifically, the electronic device according to an example embodiment may interoperate with a plurality of external electronic devices (e.g., the external electronic devices 402 and 403 of FIG. 4). The method of operating the electronic device may include operation 510 of determining an exercise starting timepoint of a user wearing the external electronic devices, based on sensor data about a heart rate acquired from at least one of the external electronic devices, operation 520 of estimating an exercise posture of the user based on sensor data about a posture of a first external electronic device, as acquired from the first external electronic device from among the interoperating external electronic devices, operation 530 of estimating a change pattern of a relative position between the external electronic devices based on the exercise starting timepoint, based on sensor data about the relative position between the external electronic devices acquired from the external electronic devices, and operation 540 of generating exercise information based on the estimated exercise posture and the estimated change pattern of the relative position between the external electronic devices.

Operation 510, according to an example embodiment, may include determining an exercise starting timepoint of a user wearing the external electronic devices, based on sensor data indicating a heart rate acquired from at least one of the external electronic devices. The sensor data indicating the heart rate may be acquired using a biometric sensor included in at least one external electronic device, that is interoperating with the electronic device. The biometric sensor may measure a biosignal (e.g., a biometric signal) of a part of a user's body (e.g., a fingertip and/or a wrist), and convert the biosignal into an electric signal. The biometric sensor may include, for example, a photoplethysmography (PPG) sensor and/or an electrocardiography (ECG) sensor. Hereinafter, embodiments in which a PPG sensor is utilized to detect heart rate will be described by way of. It is noted that this is for convenience of description, and the biometric sensor is not limited to being the PPG sensor.

The sensor data indicating the heart rate may include a heart rate waveform representing a measurements of the heart rate over time, as detected by the biometric sensor. In an example, the PPG sensor may interoperate with a light (e.g., a LED) that emits light into the tissue of a user wearing the external electronic device. The light may be reflected back towards the PPG sensor when encountering body tissue, blood, etc., and the PPG sensor may convert the detected light into into an electromagnetic value corresponding to a value indicative of an amount or quality of the detected light. The PPG sensor may therefore output a PPG waveform which may be depicted as a graph, indicative of a change in a blood flow rate over time, by generating measurement values corresponding to the biosignal over a predetermined period of time. The PPG waveform may represent an electric signal corresponding to a change in a blood flow rate between a systolic phase or a diastolic phase of a cardiac cycle. The PPG waveform may correspond to a waveform indicative of a heart rate.

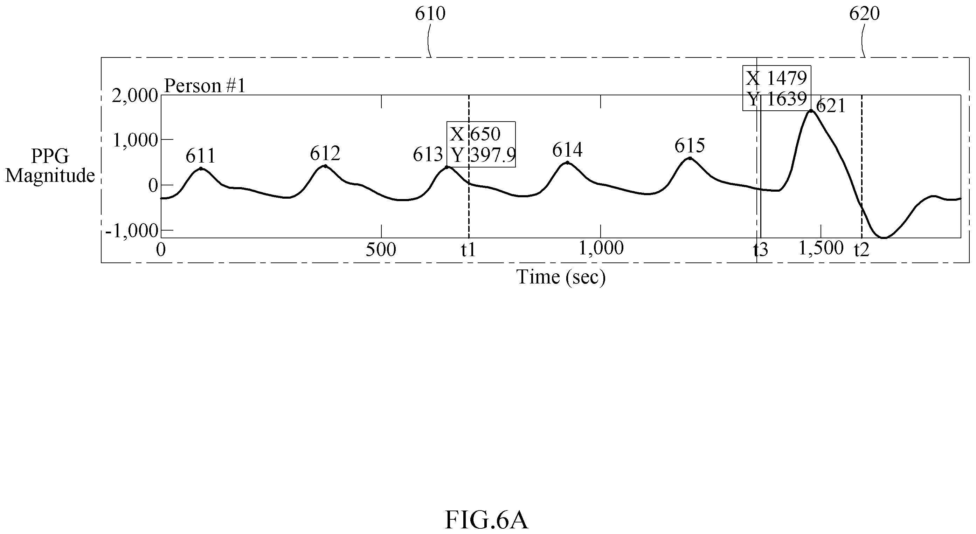

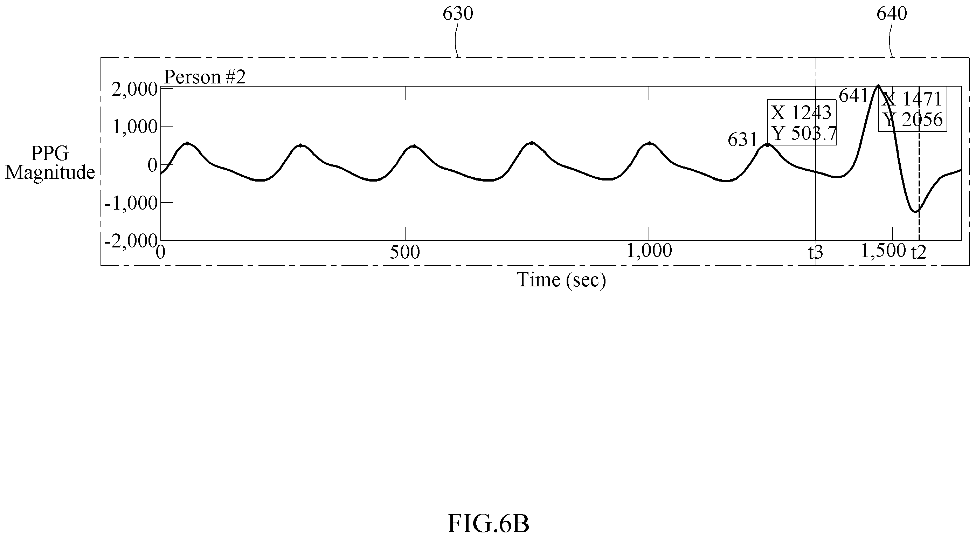

For example, FIGS. 6A and 6B illustrate PPG waveforms acquired from a first user and a second user, respectively. Referring to FIGS. 6A and 6B, in idle states 610 and 630 in which a user does not perform an exercise, a predetermined pattern of a PPG waveform is repeated. A variation of a PPG waveform in the exercise states 620 and 640 may be greater than a variation of the PPG waveform in the idle states 610 and 630. In other words, a shape of the PPG waveform in the exercise states 620 and 640 may be different from a shape of the PPG waveform in the idle states 610 and 630. Since a PPG waveform varies depending on an exercise states 620 and 640 and an idle states 610 and 630, an exercise starting timepoint may be determined based on the PPG waveform.

An electronic device according to an example embodiment may determine an exercise starting timepoint based on a ratio between peak values of a heart rate waveform. The electronic device may acquire a peak value of a heart rate waveform, based on sensor data acquired from an external electronic device. The peak value of the heart rate waveform may refer to a largest measured value in a single period within the heart rate waveform. For example, y values corresponding to points 611 to 615 and 621 of FIG. 6A may correspond to peak values.

Operation 510 may include acquiring a first peak value and a second peak value of the heart rate waveform, as based on the sensor data indicative of the heart rate, comparing a ratio between the first peak value and the second peak value to a predetermined threshold, and determining a time corresponding to the first peak value as an exercise starting timepoint based on a result of the comparison. The ratio between the first peak value and the second peak value may correspond to, for example, a ratio of the first peak value to the second peak value. Hereinafter, the ratio between the first peak value and the second peak value will be described as a ratio of the first peak value to the second peak value.

According to an example embodiment, the first peak value may include a peak value most recently acquired from the heart rate waveform. For example, in FIG. 6A, when a current time is a time t1, the first peak value may be a y value corresponding to the point 613, and when the current time is a time t2, the first peak value may be a y value corresponding to the point 621.

According to an example embodiment, the second peak value may include a value that is based on at least one peak value that was acquired before the first peak value, from the heart rate waveform. For example, the second peak value may correspond to a peak value acquired immediately before the first peak value, an average of peak values acquired before the first peak value, or a maximum value among peak values acquired before the first peak value.

In an example of a heart rate waveform of the first user shown in FIG. 6A, when the second peak value is determined as a maximum value, from among the peak values acquired before the first peak value, then a first peak value corresponding to the first user at the time t2 may be acquired as "1639" that is the y value of the point 621, and the second peak value may be acquired as "397" that is the y value of the point 613, which is the maximum value among the peak values acquired before the first peak value. In an example of a heart rate waveform of the second user shown in FIG. 6B, when the second peak value is determined as a maximum value among the peak values acquired before the first peak value, then a first peak value corresponding to the second user at the time t2 may be acquired as "2056" that is a y value of a point 641, and the second peak value may be acquired as "503.7," that is a y value of a point 631 which is the maximum value among the peak values acquired before the first peak value. A ratio "max_g/max_h" of the first peak value max_g to the second peak value max_h corresponding to each of the first user and the second user may be calculated as shown in Table 1 below.

**[Table 1]**

| | max_g | max_h | max_g/max_h |
|---|---|---|---|
| First user | 1639 | 397 | 1639/397 = 4.12 |
| Second user | 2056 | 503 | 2056/503 = 4.08 |

As described above, an amplitude and/or a period of the PPG waveform may appear to be different for each user due to various factors, even though the same exercise is performed. However, it may be found that the ratio between the first peak value and the second peak value is relatively constant.

In an example, when the ratio between the first peak value and the second peak value exceeds a predetermined threshold (e.g., "4"), a time corresponding to the first peak value may be determined to be an exercise starting timepoint. In another example, when the ratio between the first peak value and the second peak value is less than or equal to the predetermined threshold, a time corresponding to the first peak value may be determined to not be an exercise starting timepoint, and the first peak value may be used to update the second peak value. Subsequently, the first peak value may be acquired again based on sensor data about a heart rate.

For example, referring to FIG. 6A, when the current time is t1, the first peak value may correspond to the y value of the point 613, and the second peak value may correspond to a y value of either the point 611 or 612. In this example, since the ratio between the first peak value and the second peak value is calculated to be a value close to "1," which is less than or equal to the threshold (e.g., "4"), a time corresponding to the second peak value may not be determined to be the exercise starting timepoint. Subsequently, when sensor data indicative of the heart rate continues to be received from a PPG sensor of an external electronic device when some duration passes such that the current time is now t2, the first peak value may now correspond to the y value of the point 621, and the second peak value may now be updated to be the y value of the point 613. As described above with reference to Table 1, since the ratio between the first peak value and the second peak value here now exceeds "4", a time corresponding to the first peak value (i.e., 621) may be determined to be an exercise starting timepoint.

According to an example embodiment, the time corresponding to the first peak value may be determined according to a predetermined criterion based on a heart rate waveform, and may include, for example, a time at which a blood flow rate starts to rapidly increase, or a time after a predetermined amount of time elapses from the time at which a blood flow rate starts to rapidly increase. In FIGS. 6A and 6B, a time corresponding to the point 621 may be determined as t3 that is a time after a predetermined amount of time elapses from a time at which the blood flow rate increases.

Referring back to FIG. 5, operation 520 may include estimating an exercise posture of the user, based on sensor data indicative of a posture of a first external electronic device, from among the external electronic devices. The sensor data here may be acquired from the first external electronic device. For example, the first external electronic device may include an electronic device equipped with a gyro sensor and an acceleration sensor, for detecting posture (e.g., tilt, angle, disposition, etc.) and corresponding values thereof.

According to an example embodiment, the first external electronic device may be classified as a "static" electronic device. Such a device may be worn on a position of a user's body such that the posture of the static electronic device does not change significantly despite continued movement of the user. For example, devices that may be classified a static electronic devices may include wireless earphones and/or smart glasses. In contrast, "dynamic" electronic devices are typically worn where their positions may change significantly according to continued movement of the user. A typical dynamic electronic device may include, for example, a smart watch. The electronic device may determine whether each external electronic device corresponds to a static electronic device or a dynamic electronic device, based on interoperation information of external electronic devices, and may recognize which external electronic device transmits sensor data to the electronic device. Thus, the electronic device may determine whether an external electronic device that transmits the sensor data should be classified as a static electronic device or a dynamic electronic device.

According to an example embodiment, operation 520 may further include correcting (e.g., calibrating) a posture value of the first external electronic device as detected during the exercise starting timepoint, based on a surface of the earth, and estimating an exercise posture of the user based on a change in the posture value of the first external electronic device, and the corrected posture value of the first external electronic device.

According to an example embodiment, the sensor data about the posture of the first external electronic device may include yaw, pitch and roll values for the first external electronic device, as measured by a gyro sensor and/or an acceleration sensor included in the first external electronic device. In other words, the sensor data indicating the posture of the first external electronic device, as acquired from the first external electronic device, may indicate to a posture value of the first external electronic device according to a body coordinate system expressed using "Euler" angles. According to an example embodiment, the sensor data regarding the posture of the first external electronic device may include a series of posture values of the first external electronic device as detected over time.

According to an example embodiment, the posture value of the first external electronic device corresponding to the exercise starting timepoint may be acquired using the gyro sensor and/or the acceleration sensor of the first external electronic device, during the exercise starting timepoint determined in operation 510. If an input to set an initial posture of an exercise is received from a user, the posture value of the first external electronic device may refer to a posture value of the first external electronic device acquired based on the gyro sensor and/or the acceleration sensor of the first external electronic device at a time where the input is received from the user.

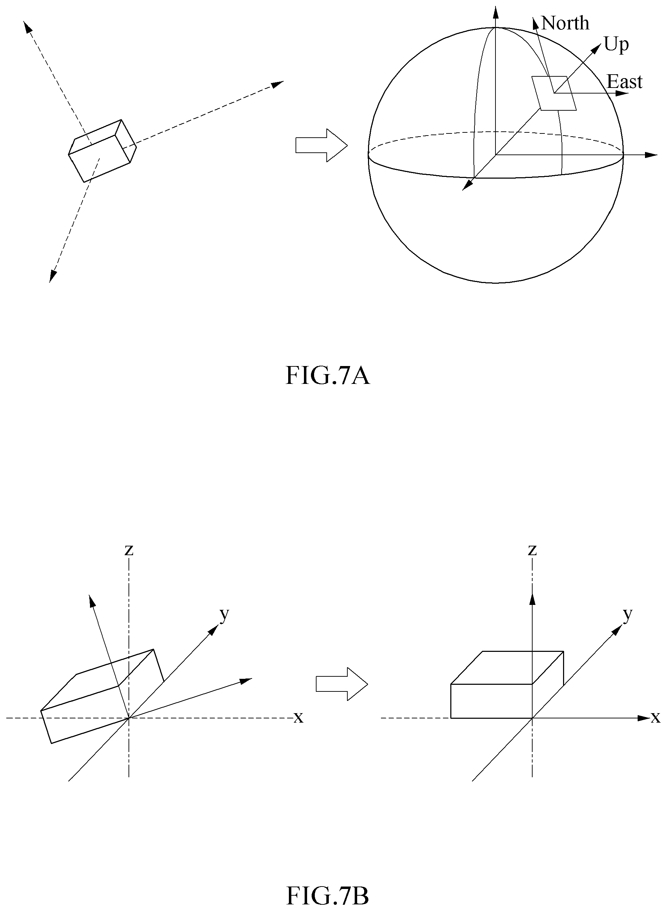

According to an example embodiment, the posture value of the first external electronic device corresponding to the exercise starting timepoint may be corrected (e.g., calibrated) based on the surface of the earth. For example, referring to FIG. 7A, a posture value of a first external electronic device according to the body coordinate system indicating a degree of rotation of the first external electronic device about three axes may be corrected based on a "North East Up" (NEU) coordinate system centered on the surface of the earth.

More specifically, the correcting of the posture value of the first external electronic device based on the surface of the earth may include setting the roll and a pitch of the first external electronic device to values parallel to the surface of the earth and perpendicular to a direction of gravity. For example, referring to FIG. 7B, with posture values of the first external electronic device include yaw, pitch, and roll, the pitch value and the roll value indicating horizontal inclination may be corrected to be parallel to an x-y plane according to the NEU coordinate system. Based on the corrected posture value of the first external electronic device, the posture value of the first external electronic device (e.g., corresponding to the exercise starting timepoint) may be recognized as being parallel to the surface of the earth.

According to an example embodiment, operation 520 of estimating the exercise posture may include acquiring a direction in which the posture of the first external electronic device is changed and a degree to which the posture of the first external electronic device is changed, based on sensor data about the posture of the first external electronic device acquired after the exercise starting timepoint, and estimating a changed exercise posture based on an initial posture corresponding to the exercise starting timepoint, based on the direction and the degree. The initial posture corresponding to the exercise starting timepoint may be set manually via a setting provided a user, or be set to a preset default value. The initial posture may be set to, for example, one of standing perpendicular to the surface of the earth, a lying flat parallel to the surface of the earth, and lying flat and face down parallel to the surface of the earth.

According to an example embodiment, a posture value of the first external electronic device, as acquired from a sensor of the first external electronic device, may change according to a movement of a user wearing the first external electronic device. The sensor data regarding the posture of the first external electronic device may include posture values of the first external electronic device that are acquired over time. The direction in which the posture of the first external electronic device is changed, and the degree to which the posture of the first external electronic device is changed may be acquired based on a difference between a posture value of the first external electronic device acquired at the exercise starting timepoint, and a posture value of the first external electronic device acquired after the exercise starting timepoint is concluded, within the sensor data regarding the posture of the first external electronic device. The changed exercise posture of the user may be estimated based on an initial posture of the user corresponding to the exercise starting timepoint, based on the direction and the degree.

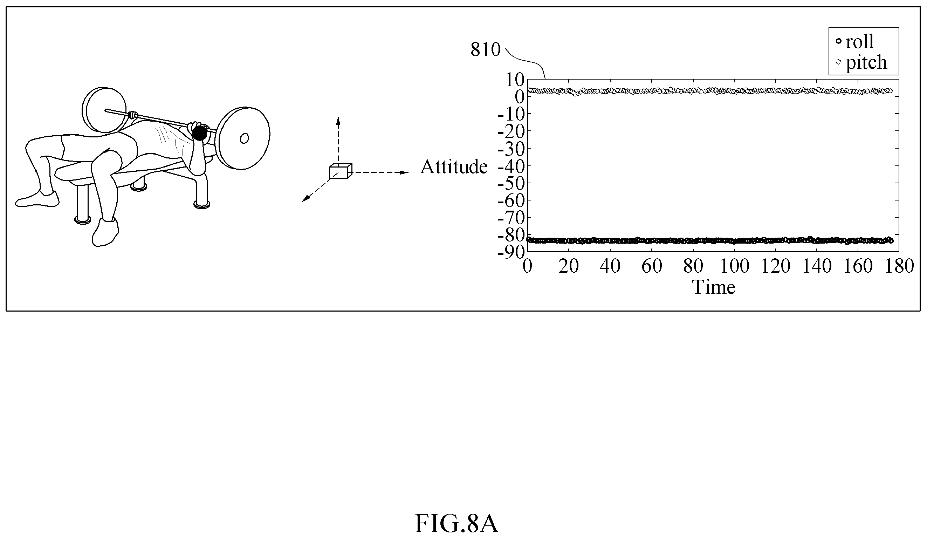

For example, FIGS. 8A to 8C are diagrams illustrating posture values of a first external electronic device, according to an exercise posture of a user. The first external electronic device may correspond to a wearable device (e.g., a wireless earphone, and glasses) worn on a head portion (e.g., an ear, and a face) of the user.

Referring to FIG. 8A, a posture value of the first external electronic device corresponding to an exercise starting timepoint may be acquired as a value corresponding to a graph 810, and an initial posture of a user corresponding to the exercise starting timepoint may be set to a posture of lying parallel to the surface of the earth. In other words, an electronic device may recognize a posture value of the first external electronic device corresponding to the graph 810 received from the first external electronic device as a value corresponding to the posture of lying flat parallel to the surface of the earth. According to an example embodiment, to recognize the received posture value of the first external electronic device corresponding to the exercise starting timepoint as a value of the posture of lying parallel to the surface of the earth, the electronic device may correct the roll and pitch values of the first external electronic device to be parallel to the surface of the earth, and correct a next received posture value of the first external electronic device in the same manner. By correcting the posture value of the first external electronic device, an exercise posture of a user may be estimated regardless of how the first external electronic device is worn. In other words, even if the user wears the first external electronic device such that the device is disposed in an inclined state during a time in which the exercise is performed, the posture value of the first external electronic device corresponding to the exercise starting timepoint may be corrected to indicate a state of being parallel to the surface of the earth, and the exercise posture may be accurately estimated as a result.

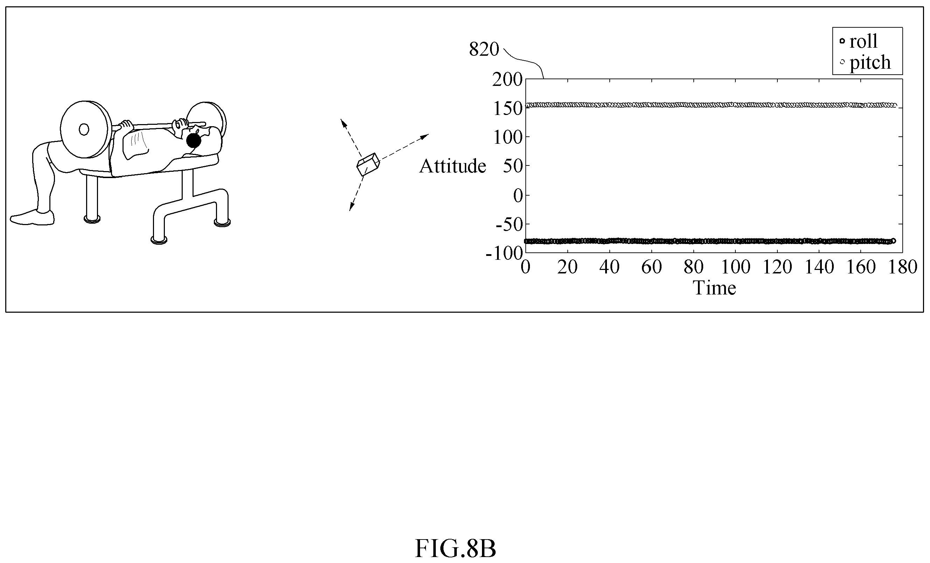

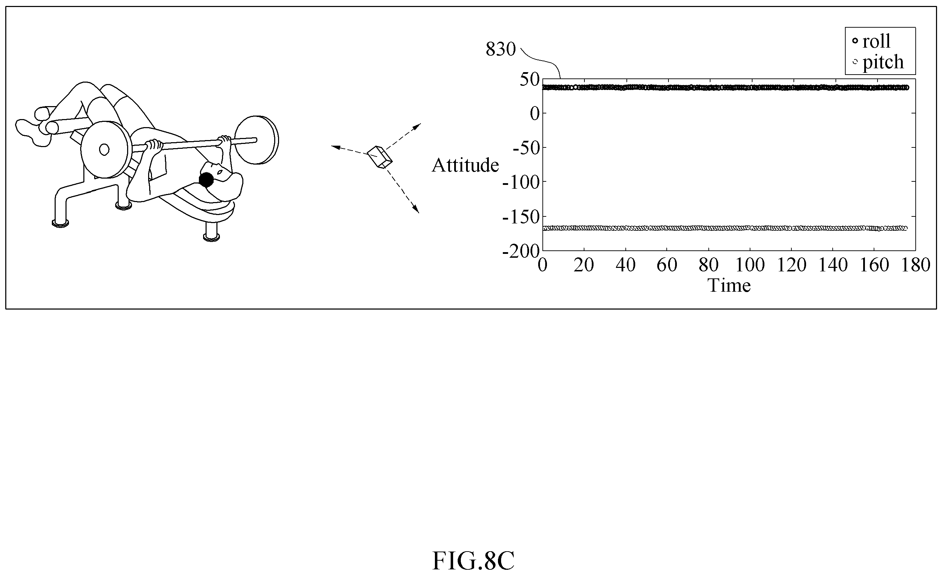

Referring to FIG. 8B, when the exercise posture of the user is changed into an inclined position after the exercise starting timepoint, a head of the user may be tilt, and accordingly, a posture value of the first external electronic device (i.e., if worn on an ear of the user) may be changed to the values indicated in the corresponding graph 820. In addition, referring to FIG. 8C, when the exercise posture of the user is changed to a declined position after the exercise starting timepoint, the head of the user may again be tilted, and accordingly the posture value of the first external electronic device worn on the ear of the user may be changed to the values indicated in the corresponding graph 830.

According to an example embodiment, a direction in which the posture value of the first external electronic device is changed, and a degree to which the posture value of the first external electronic device is changed may be acquired based on a difference between a posture value of the first external electronic device acquired at the exercise starting timepoint, and a posture value of the first external electronic device acquired after the exercise starting timepoint. A change in the exercise posture of the user may be estimated based on an initial posture according to the direction, and the degree of change. For example, based on the posture value of the first external electronic device corresponding to the exercise starting timepoint, and the posture value of the first external electronic device changed after the exercise starting timepoint, changes in the exercise posture of the user may be determinable (e.g., flat to incline, incline to decline, etc.).

Referring back to FIG. 5, operation 530 of estimating the change pattern of the relative position between the external electronic devices, according to an example embodiment, may include estimating the change pattern of the relative position between the external electronic devices based on the exercise starting timepoint, based on sensor data about the relative position between the external electronic devices acquired from the external electronic devices.

According to an example embodiment, operation 530 may include monitoring the distance between the two external electronic devices over the time period after the exercise starting timepoint. Monitoring may be executed using sensor data that is acquired from the external electronic devices (e.g., including data indicative of relative distance), and then estimating, based on the sensor data indicating distance over time, whether a pattern indicated by the changes in distance between the two devices corresponds to a preset known pattern. The preset pattern may indicate an increase in distance, followed by a decrease in the distance. Alternatively, the preset pattern may indicate a decrease in distance, followed by an increase in the distance.. The sensor data indicative of changes in the distance may be acquired using a distance sensor included in an external electronic device interoperating with the electronic device. The distance sensor may measure a distance to another object using, for example, time-of-flight calculations via generated ultrasonic waves or rays transmitted from the sensor to the object, that are then reflected by the object and detected upon return. The distance sensor may include, for example, an ultrasonic sensor, an infrared sensor, a lidar sensor, a radar sensor, an ultra wideband (UWB) sensor, and/or a time of flight (TOF) sensor.

According to an example embodiment, a distance sensor included in a predetermined external electronic device may measure a distance to another external electronic device, generate sensor data about the distance, and output the sensor data indicative of the distance, as continually measured over a certain time. When the distance between the above two external electronic devices is acquired at an exercise starting timepoint based on the sensor data about the distance, a pattern of changes in the distance may be detected. For example, the distance between the external electronic devices may increase and then decrease, or decrease and then increase. Thus, a pattern indicative of changes in relative positions and distance between the external electronic devices may be estimated.

According to an example embodiment, exercise types may be classified according to whether the estimated change pattern in distance between the external electronic devices indicates initial increase followed by decrease, or initial decrease followed by increase. In an example, when a pattern in which a distance between external electronic devices acquired at the exercise starting timepoint increases and then decreases after the exercise starting timepoint is repeated, a type of exercise performed by a user may be classified as a type of "pushing" exercise. In another example, when a pattern in which the distance between the external electronic devices acquired at the exercise starting timepoint decreases and then increases after the exercise starting timepoint is repeated, the type of exercise performed by the user may be classified as a type of "pulling" exercise.

According to an example embodiment, operation 530 of estimating the pattern of change in distance (e.g., in the relative positions) between each of the external electronic devices may further include estimating a relative position between the external electronic devices, according to a height difference between the external electronic devices, based on sensor data about a height (e.g., altitude) acquired from each of the external electronic devices. The sensor data about the height may be acquired based on an atmospheric pressure sensor included in an external electronic device. The atmospheric pressure sensor may detect atmospheric pressure, and may estimate a height of a sensor based on the relationship between altitude and atmospheric pressure, which decreases as altitude increases. The relative position between the external electronic devices, accounting for a height difference between the external electronic devices, may be acquired based on the sensor data indicating the height (e.g., relative to a surface of the earth), as acquired from each of the external electronic devices.

Operation 530 may include estimating a change pattern of a movement direction for each of the external electronic devices, based on sensor data indicating movement directions acquired from each of the external electronic devices. The sensor data about the movement direction may be acquired based on an inertial sensor included in an external electronic device. The inertial sensor may measure a specific force, an angular ratio, and/or a magnetic field, and may include, for example, an acceleration sensor, a gyro sensor, and/or a geomagnetic sensor. A change pattern in movement direction of an external electronic device, for example, may indicate whether the external electronic device is moving vertically or horizontally and/or rotating, may be estimated based on sensor data indicative of the movement direction as acquired from the external electronic device.

Operation 540 may include generating the exercise information based on the estimated exercise posture and the estimated change pattern of the relative position between the external electronic devices.

According to an example embodiment, operation 540 may include generating exercise information, including a detailed posture of exercise, as based on an inclination of the estimated exercise posture. The information on the detailed posture of the exercise may include information on a type of exercise, as classified into detailed postures according to the inclination of the exercise posture. The inclination of the exercise posture may include an inclination of a body of a user with respect to a horizontal plane or an inclination changed based on the inclination of the body of the user at an exercise of the user.

For example, referring to FIGS. 8A to 8C, a bench press may be classified so as to include detailed postures of a flat bench press, an incline bench press, and a decline bench press, according to the inclination of the exercise posture. The electronic device according to an example embodiment may generate information on whether a posture of a user performing the bench press exercise corresponds to the incline bench press, the flat bench press, or the decline bench press, based on the inclination of the exercise posture of the user that was previously estimated in operation 520.

The type of exercise performed by the user according to an example embodiment may also be input to the electronic device by the user, or may be estimated based on received sensor data in the electronic device. An operation of estimating the type of exercise performed by the user based on received sensor data in the electronic device will be further described below. The electronic device may receive sensor data from an external electronic device based on an input type of an exercise and may provide the user with information on a detailed posture of the exercise according to an inclination of an exercise posture.

According to an example embodiment, operation 540 may include generating information on a number of repetitions of an exercise performed the user, based on the estimated change pattern of the relative position between the external electronic devices. The electronic device may estimate the number of repetitions of the exercise performed by the user, based on a number of repetitions of changes indicated in the change pattern (e.g., previously estimated in operation 530). For example, when the estimated distance between external electronic devices shows a pattern in which decreasing and then increasing is repeated ten times, it may be estimated that the has performed ten repetitions of a corresponding exercise, such as a push-up. As described above, the type of the exercise performed by the user may be input to the electronic device by the user, or may be estimated based on the sensor data received from the electronic device.

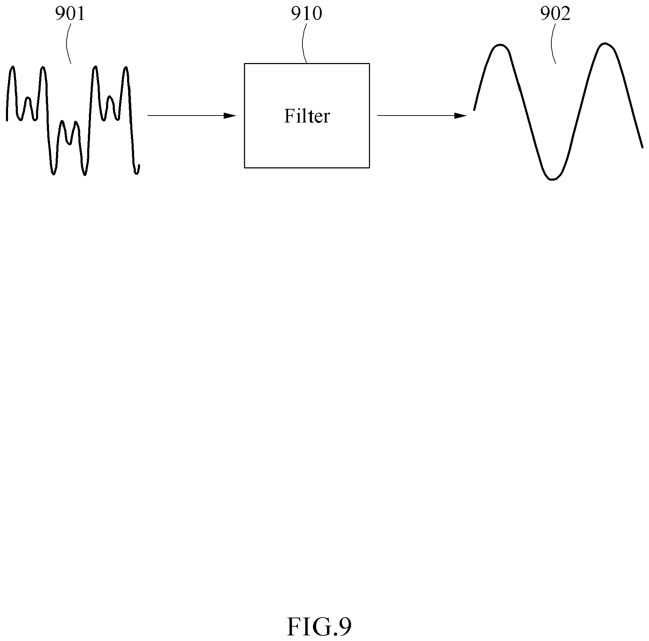

According to an example embodiment, the number of repetitions of the exercise may be estimated based on a pattern of a signal included in the sensor data. The pattern of the signal included in the sensor data may be converted into a signal with a pattern countable by a signal processing scheme. The signal processing scheme may include, for example, a filtering scheme. In an example, referring to FIG. 9, a pattern 901 for a signal included in sensor data may be converted to a signal 902 having a predetermined pattern based on a filter 910that is designed to pass a desired portions or fluctuations in a waveform and filter out an unwanted portions in the waveform (e.g., a filter that passes only about a 2 hertz "Hz" frequency signals).

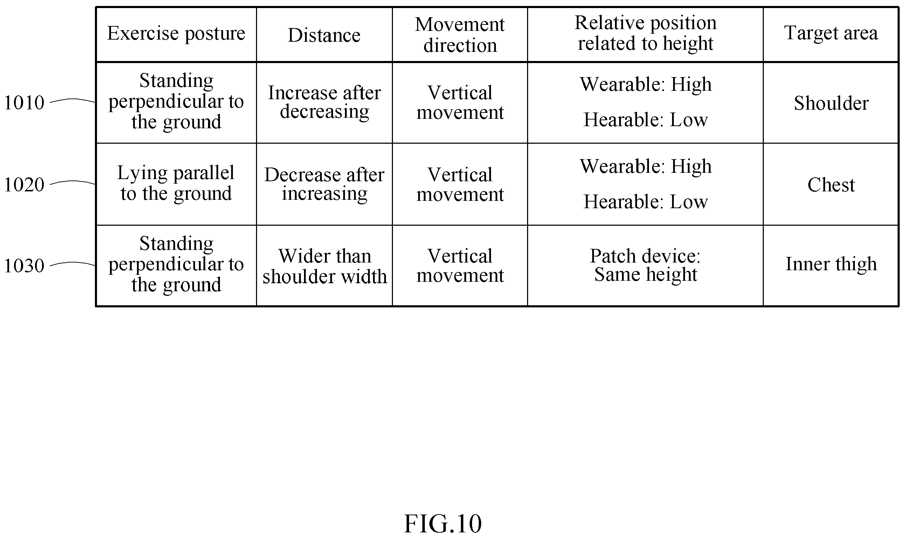

Referring back to FIG. 5, operation 540 may further include generating information on a target area, based on the exercise posture, the change pattern of the distance between the external electronic devices, the relative position according to the height difference between the external electronic devices, and the change pattern of the movement direction of each of the external electronic device. For example, FIG. 10 illustrates examples of target areas corresponding to an exercise posture, a change pattern of a distance between external electronic devices, a relative position according to a height difference between the external electronic devices, and a change pattern of a movement direction of each of the external electronic device, when a wearable device worn on a wrist and a hearable device worn on an ear are included as external electronic devices. In an example, as shown in a first row 1010 of a table of FIG. 10, when the exercise posture estimated in operation 520 of FIG. 5 is determined to be standing perpendicular to the ground, when the change pattern of the distance between the external electronic devices estimated in operation 530 of FIG. 5 is determined to be a change pattern in which the distance increases and then decreases, when the change pattern for the movement direction of each of the external electronic devices is determined as a vertical exercise, and when the relative position according to the height difference between the external electronic devices indicates that the wearable device (e.g., a smartwatch) is located above the other wearable device (e.g., a "hearable" device, such as an earphone), an electronic device may determine that a user is performing a "lat" pulldown exercise, and generate information indicating that the target area is the shoulder region of the body. In another example, in a second row 1020 of the table of FIG. 10, the electronic device may determine that the user is perform a bench press exercise, and may generate information indicating that the target area corresponds to a chest region of the body. In another example, in a third row 1030 of the table of FIG. 10, the electronic device may determine that the user is performing a wide squat, and may generate information indicating that the target area corresponds to an inner thigh region of the body.

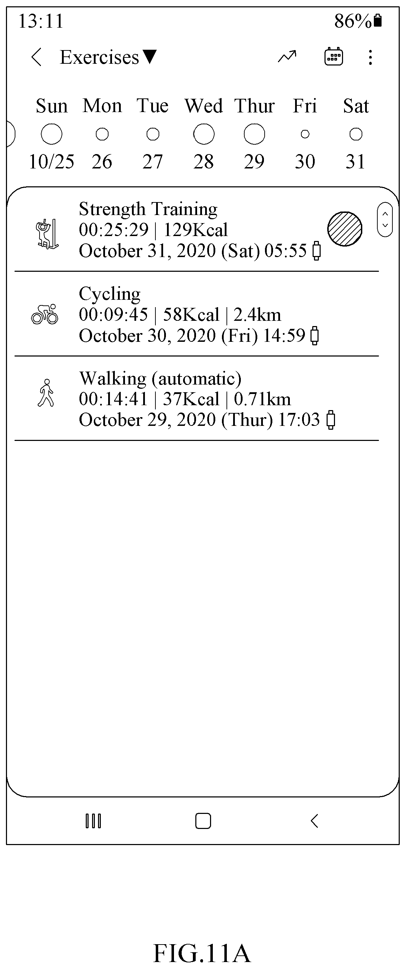

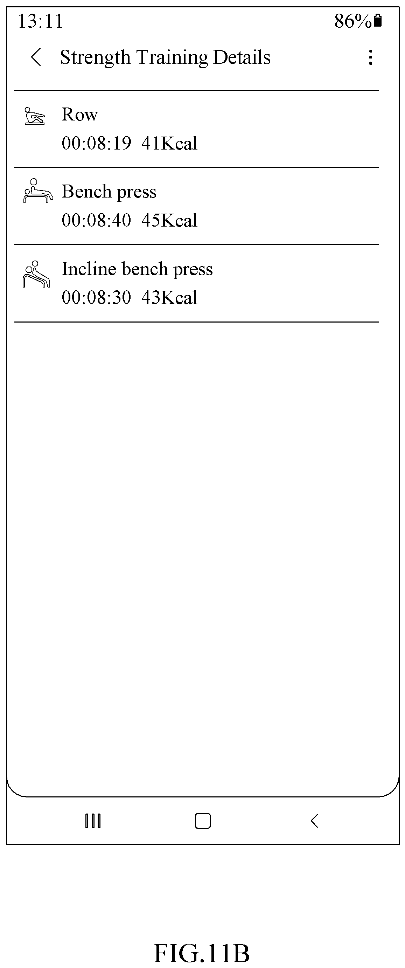

Referring back to FIG. 5, the exercise information generated in operation 540 according to an example embodiment may be output through an output module such as a display and/or a speaker of the electronic device. For example, the exercise information generated in operation 540 may be provided to a user through an interface displayed on a display of an electronic device shown in FIGS. 11A and 11B. Referring to FIGS. 11A and 11B, exercises performed by a user may be displayed as a single exercise item (e.g., strength training), and the user may select a corresponding exercise item and check information. Exercise information of an exercise item may include calories burned according to a type of an exercise, an exercise duration, and a number of repetitions of an exercise. As described above, the exercise information may include information on a detailed posture (e.g., a bench press, and an incline bench press) of an exercise according to an estimated exercise posture. The information on the detailed posture of the exercise may include information on the number of repetitions of the exercise, the exercise duration, and calories burned by performing the exercise which correspond to the detailed posture of the exercise.

Although not shown in FIGS. 11A and 11B, according to an example embodiment, when detailed postures of an exercise fail to be identified and analyzed, results of an exercise performed by the user may be grouped and displayed as a single item (e.g., strength training), instead of being classified into items according to the detailed postures. Alternatively, exercises in which detailed postures may be analyzed may be displayed by items, and exercises in which detailed postures fail to be identified may be grouped and displayed as a single item (e.g., strength training).

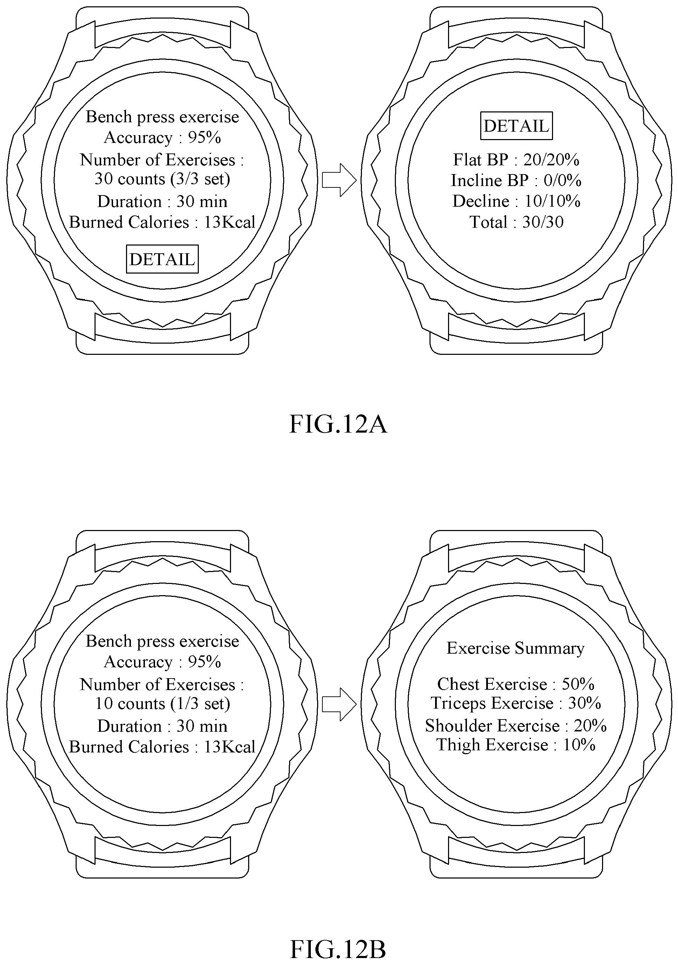

Referring back to FIG. 5, the exercise information generated in operation 540 according to an example embodiment may be transmitted to an external electronic device interoperating with the electronic device, and may be provided to a user through an output module of the external electronic device. For example, the exercise information generated in operation 540 may be provided to a user through an interface displayed on a display of a smart watch that is an external electronic device interoperating with an electronic device shown in FIGS. 12A and 12B. Referring to FIG. 12A, the exercise information provided to the user may include information on a detailed posture (e.g., a decline bench press, and an incline bench press) of an exercise according to an estimated exercise posture. Referring to FIG. 12B, the exercise information provided to the user may include information on a target area. The information on the target area may include an exercise percentage of each target area.

An electronic device (e.g., the electronic device 101 of FIG. 1 or the electronic device 401 of FIG. 4) according to an example embodiment may include a communication module (e.g., the communication module 190 of FIG. 1 or the communication module 190 of FIG. 4) configured to communicate with a plurality of external electronic devices (e.g., the external electronic devices 402 and 403 of FIG. 4) that are wearable on a body of a user, at least one processor (e.g., the processor 120 of FIG. 1 or the processor 120 of FIG. 4) configured to determine an exercise starting timepoint of a user wearing the external electronic devices based on sensor data about a heart rate acquired from at least one of the external electronic devices, to estimate an exercise posture of the user based on sensor data about a posture of a first external electronic device among the external electronic devices acquired from the first external electronic device, to estimate a change pattern of a relative position between the external electronic devices based on the exercise starting timepoint, based on sensor data about the relative position between the external electronic devices acquired from the external electronic devices, and to generate exercise information based on the estimated exercise posture and the estimated change pattern of the relative position between the external electronic devices, and an output module (e.g., the input module 150, the sound output module 155, and the display module 160 of FIG. 1, or the I/O module 410 of FIG. 4) configured to output the generated exercise information.

The communication module according to an example embodiment may communicate with an external electronic device interoperating with the electronic device, and may receive, for example, sensed data from a sensor of the external electronic device. In addition, data generated or processed by the electronic device may be transmitted to the external electronic device, and data received from another external electronic device may be transmitted to the external electronic device.

The processor according to an example embodiment may be operatively connected to an I/O module, a communication module, and a memory, and may perform an operation of the electronic device. For example, the processor may receive sensed data from at least one external electronic device worn by a user of the electronic device who is performing an exercise, and may generate exercise information corresponding to the user. An operation of the electronic device to generate the exercise information using the processor may be the same as that described above with reference to FIG. 5.

An I/O module according to an example embodiment may include an input module (e.g., the input module 150 of FIG. 1) and an output module (e.g., the sound output module 155 and/or the display module 160 of FIG. 1). The output module may be a module configured to display information generated by the processor to a user, and may include, for example, a display.

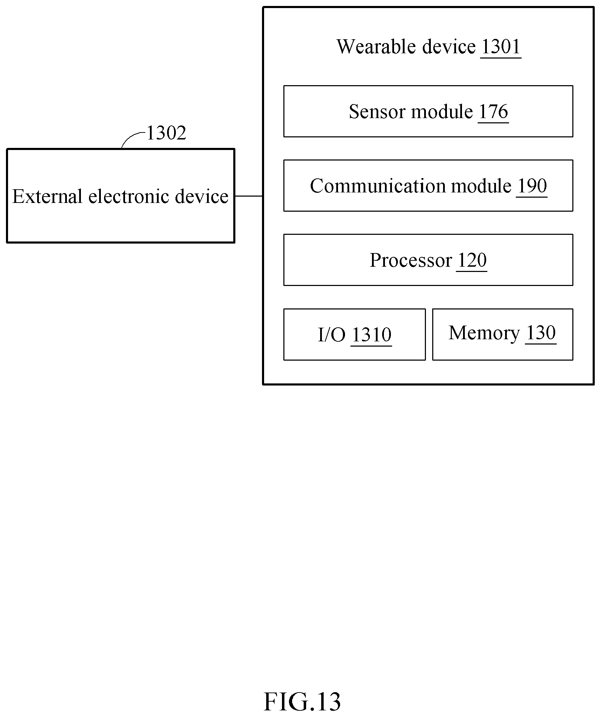

FIG. 13 is a block diagram of a wearable device interoperating with an external electronic device according to certain example embodiments.

Referring to FIG. 13, a wearable device 1301 (e.g., the electronic device 101 of FIG. 1, the electronic device 200 of FIG. 2A, the electronic device 200 of FIG. 2B, or the electronic device 300 of FIG. 3) according to an example embodiment may include a sensor module 176 (e.g., the sensor module 176 of FIG. 1), a communication module 190 (e.g., the communication module 190 of FIG. 1), a processor 120 (e.g., the processor 120 of FIG. 1), an I/O module 1310 (e.g., the input module 150, the sound output module 155, and/or the display module 160 of FIG. 1), and a memory 130 (e.g., the memory 130 of FIG. 1), and may interoperate with at least one external electronic device 1302 (e.g., the first external electronic device 402 of FIG. 4 or the second external electronic device 403 of FIG. 4). The wearable device 1301 may include, for example, a smart watch. The wearable device 1301 may further include the configuration of the electronic device 101 described above with reference to FIG. 1 in addition to the configuration shown in FIG. 13. The wearable device 1301 according to an example embodiment may interoperate with other electronic devices (e.g., the electronic device 101 of FIG. 1 or the electronic device 401 of FIG. 4) as well as the external electronic device 1302.

The wearable device 1301 according to an example embodiment may interoperate with the at least one external electronic device 1302, and may include the sensor module 176 including at least one sensor, at least one processor 120, and the I/O module 1310. The at least one processor 120 may be configured to determine an exercise starting timepoint of a user wearing the wearable device 1301 based on sensor data about a heart rate corresponding to the user sensed in the sensor, to estimate an exercise posture of the user based on sensor data about a posture of the wearable device 1301 sensed in the sensor, to estimate a change pattern of a relative position with the external electronic device 1302 based on the exercise starting timepoint, based on sensor data about the relative position with the external electronic device 1302 sensed in the sensor, and to generate exercise information based on the estimated exercise posture and the estimated change pattern of the relative position with the external electronic device 1302. The I/O module 1310 may be configured to output the generated exercise information.

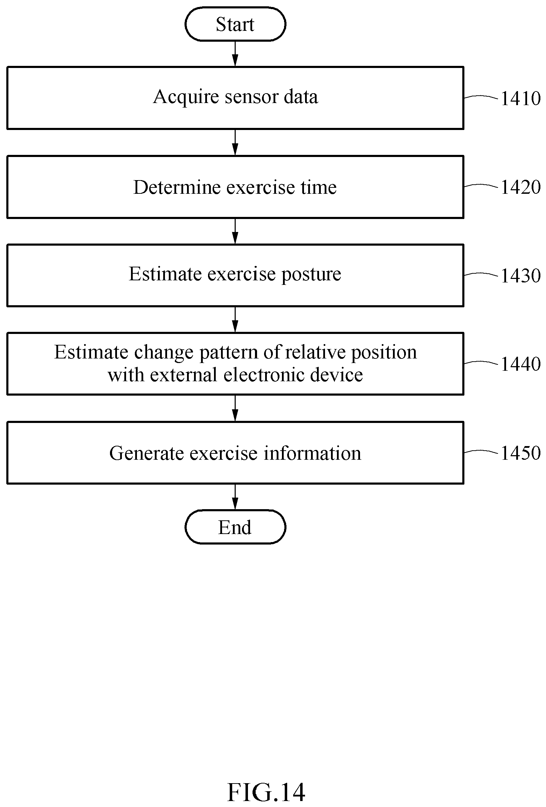

FIG. 14 is a flowchart illustrating a method of operating a wearable device according to certain example embodiments.

Referring to FIG. 14, a method of operating a wearable device (e.g., the electronic device 101 of FIG. 1, the electronic device 200 of FIG. 2A, the electronic device 200 of FIG. 2B, the electronic device 300 of FIG. 3, or the electronic device 1301 of FIG. 13) according to an example embodiment may include operation 1410 of acquiring sensor data, operation 1420 of determining an exercise starting timepoint, operation 1430 of estimating an exercise posture, operation 1440 of estimating a change pattern of a relative position with an external electronic device, and operation 1450 of generating exercise information. The method of FIG. 14 may be performed by, for example, a processor (e.g., the processor 120 of FIG. 1 or the processor 120 of FIG. 13) of the electronic device.

More specifically, the wearable device according to an example embodiment may interoperate with at least one external electronic device (e.g., the external electronic device 402 or 403 of FIG. 4 or the external electronic device 1302 of FIG. 13). The method of operating the wearable device may include acquiring sensor data based on at least one sensor included in the wearable device and at least one sensor included in the external electronic device, determining an exercise starting timepoint of a user wearing the wearable device, based on sensor data about a heart rate corresponding to the user, estimating an exercise posture of the user based on sensor data about a posture of the wearable device, estimating a change pattern of a relative position with the external electronic device based on the exercise starting timepoint, based on sensor data about the relative position with the external electronic device, and generating exercise information based on the estimated exercise posture and the estimated change pattern of the relative position with the external electronic device.

The wearable device according to an example embodiment may acquire sensor data from a sensor included in the wearable device as well as a sensor included in the external electronic device interoperating with the wearable device. The method of FIG. 5 and the method of FIG. 14 may differ from each other in that the method of FIG. 5 includes operations of using sensor data acquired from at least one of the external electronic devices interoperating with the electronic device and the method of FIG. 14 includes an operation of using sensor data acquired from a sensor included in the wearable device. Operations 1420 to 1450 of the wearable device according to an example embodiment may correspond to operations 510 to 540 of FIG. 5, except for a difference in whether sensor data acquired from another device is used or sensor data acquired from a sensor included in the wearable device is used.

According to an example embodiment, exercise information generated by the wearable device may be transmitted to an electronic device (e.g., a smartphone) of a user interoperating with the wearable device for continuity of use or to a server that stores existing exercise information corresponding to the user, and the transmitted exercise information may be displayed using an output module (e.g., a display) of the electronic device interoperating the wearable device. According to an example embodiment, the wearable device that generates and stores the exercise information, the electronic device of the user interoperating with the wearable device, and the server that stores the exercise information corresponding to the user may be periodically synchronized, so that the exercise information generated in one device may be updated on another device and that the same exercise information may be provided to the user.

## Claims

1. A method of operating an electronic device (401) interoperating with a plurality of external electronic devices (402, 403) that are wearable on a body of a user, the method comprising:
receiving, via a communication circuit (190) from the plurality of external electronic devices, first sensor data including a heart rate of the user, second sensor data indicating a posture of a first external electronic device from among the plurality of external electronic devices, and third sensor data indicating relative position between the plurality of external electronic devices;
determining (510), via at least one processor (120), an exercise starting timepoint based on the first sensor data;
estimating (520) an exercise posture of the user based on the second sensor data;
estimating (530) a pattern of change in distance between each of the plurality of external electronic devices, indicated by changes in the relative position between each the plurality of external electronic devices over a time period after the determined exercise starting timepoint, and based on the third sensor data; and
generating (540) exercise information based on the estimated exercise posture and the estimated pattern of change in distance between the plurality of external electronic devices.

2. The method of claim 1, wherein the determining of the exercise starting timepoint comprises:
detecting a first peak value and a second peak value of a heart rate based on the first sensor data;
comparing a ratio between the first peak value and the second peak value to a predetermined threshold; and
determining a timepoint corresponding to the first peak value as an exercise starting timepoint, based on a result of the comparing.

3. The method of claim 2, wherein the first sensor data includes a waveform indicative of changes of the heart rate over time;
wherein the first peak value includes a most-recent peak indicated in the waveform, and
wherein the second peak value includes at least one peak value acquired prior to acquisition of the first peak value, as indicated in the waveform.

4. The method of claim 1, wherein the estimating of the exercise posture further comprises:
correcting the posture of the first electronic device indicated by the second sensor data based on calibration against a surface of the earth; and
wherein the exercise posture is estimated based on a change in value of the posture of the first external electronic device caused by correction based on calibration against the surface of the earth.

5. The method of claim 4, wherein correcting the posture of the first external electronic device further comprises: configuring roll and pitch values indicated by the second sensor data to be parallel to the surface of the earth, and perpendicular to gravity.

6. The method of claim 1, wherein estimating the exercise posture comprises:
for a time period after the determined exercise starting timepoint, detecting a change in the posture of the first electronic device, including a change in roll and/or pitch, and a degree of the change, as indicated in the second sensor data; and
estimating a changed exercise posture from an initial posture indicated during the exercise starting timepoint, based on the detected change in the posture.

7. The method of claim 6, wherein the initial posture includes at least one of:
standing upright perpendicular to a surface of the earth, lying flat parallel to the surface of the earth, and lying face-down parallel to the surface of the earth.

8. The method of claim 1, wherein detecting the pattern of change in distance between the plurality of external electronic devices further includes:
determining whether the pattern indicates that the distance between the plurality of external electronic devices increases and then decreases over the time period after the determined exercise starting timepoint, or whether the pattern indicates that the distance between the plurality of external electronic devices decreases and then increases over the time period after the determined exercise starting timepoint.

9. The method of claim 1, wherein the generated exercise information further includes at least one of:
a user posture for an exercise based on an inclination indicated by the estimated exercise posture; and
a number of repetitions for an exercise to be performed the user, based on the estimated pattern of change in distance between the plurality of external electronic devices.

10. The method of claim 1, wherein further comprises at least one of:
estimating a relative position between each of the plurality external electronic devices according to a height difference between each of the plurality of external electronic devices, based on sensor data indicative of respective heights of each of the plurality of external electronic devices relative to a surface of the earth, and
estimating a second pattern of change based on fourth sensor data acquired from each of the plurality of external electronic devices, respectively indicating movement directions of each of the plurality of external electronic devices.

11. The method of claim 10, wherein the exercise information is generated for a target area, indicating a region of a user's body targeted by the exercise, based one or more of:
the estimated exercise posture, the estimated pattern of change in the distance between the plurality of external electronic devices, the estimated relative position between the external electronic devices according to the height difference, and the estimated second pattern of change indicating the movement direction of each of the plurality of external electronic devices.

12. An electronic device (401), comprising:
a communication circuit (190) configured to communicate with a plurality of external electronic devices (402, 403) that are wearable on a body of a user and receive first sensor data, second sensor data, and third sensor data from the plurality of external electronic devices;
at least one processor (120) configured to:
determine an exercise starting timepoint based on the first sensor data including a heart rate of the user,
estimate an exercise posture of the user based on the second sensor data indicating a posture of a first external electronic device from among the plurality of external electronic devices,
estimate a pattern of change in distance between each of the plurality of external electronic devices, indicated by changes in a relative position between each the plurality of external electronic devices over a time period after the determined exercise starting timepoint, and based on the third sensor data indicating relative position between the plurality of external electronic devices, and
generate exercise information based on the estimated exercise posture and the estimated pattern of change in distance between the plurality of external electronic devices.

13. The electronic device of claim 12, wherein determining the exercise starting timepoint comprises:
detecting a first peak value and a second peak value of a heart rate based on the first sensor data;
comparing a ratio between the first peak value and the second peak value to a predetermined threshold; and
determining a timepoint corresponding to the first peak value as an exercise starting timepoint, based on a result of the comparing.

14. The electronic device of claim 12, wherein the estimating of the exercise posture further comprises:
correcting the posture of a first external electronic device from among the plurality of external electronic devices, as indicated by the second sensor data based on calibration against a surface of the earth; and
wherein the exercise posture is estimated based on a change in value of the posture of the first external electronic device caused by correction based on calibration against the surface of the earth.

15. A wearable device (1301) interoperating with at least one external electronic device (1302), the wearable device comprising:
a plurality of sensors; and
an output module;
at least one processor (120) configured to:
determine an exercise starting timepoint of a user wearing the wearable device based on first sensor data detected via the plurality of sensors, including a heart rate of the user,
estimate an exercise posture of the user based on second sensor data indicating a posture of the wearable device as detected by the plurality of sensors,
estimate a pattern of change in distance with the external electronic device based on the exercise starting timepoint, and on third sensor data detected by the plurality of sensors monitoring distance with the external electronic device over at least a time period after the exercise starting timepoint,
generate exercise information based at least on the estimated exercise posture, and the estimated pattern of change in the distance with the external electronic device, and
output the generated exercise information via the output module.

## Patentansprüche

1. Verfahren zum Betreiben einer elektronischen Vorrichtung (401), die mit einer Vielzahl von externen elektronischen Vorrichtungen (402, 403) zusammenarbeitet, die an einem Körper eines Benutzers tragbar sind, das Verfahren umfassend:
Empfangen über eine Kommunikationsschaltung (190) aus der Vielzahl von externen elektronischen Geräten von ersten Sensordaten, die eine Herzfrequenz des Benutzers einschließen, von zweiten Sensordaten, die eine Haltung eines ersten externen elektronischen Geräts aus der Vielzahl von externen elektronischen Geräten angeben, und von dritten Sensordaten, die eine relative Position zwischen der Vielzahl von externen elektronischen Geräten angeben;
Bestimmen (510) über zumindest einen Prozessor (120) eines Übungsstartzeitpunkts basierend auf den ersten Sensordaten;
Schätzen (520) einer Übungshaltung des Benutzers basierend auf den zweiten Sensordaten;
Schätzen (530) eines Musters der Abstandsänderung zwischen jeder der Vielzahl von externen elektronischen Vorrichtungen, angegeben durch Änderungen der relativen Position zwischen jeder der Vielzahl von externen elektronischen Vorrichtungen über einen Zeitraum nach dem bestimmten Übungsstartzeitpunkt und basierend auf den dritten Sensordaten; und
Erzeugen (540) von Übungsinformationen basierend auf der geschätzten Übungshaltung und des geschätzten Musters der Abstandsänderung zwischen der Vielzahl von externen elektronischen Vorrichtungen.

2. Verfahren nach Anspruch 1, wobei das Bestimmen des Übungsstartzeitpunkts Folgendes umfasst:
Erkennen eines ersten Spitzenwerts und eines zweiten Spitzenwerts einer Herzfrequenz basierend auf den ersten Sensordaten;
Vergleichen eines Verhältnisses zwischen dem ersten Spitzenwert und dem zweiten Spitzenwert mit einem vorbestimmten Schwellenwert; und
Bestimmen eines Zeitpunkts, der dem ersten Spitzenwert entspricht, als einen Übungsstartzeitpunkt basierend auf einem Ergebnis des Vergleichens.

3. Verfahren nach Anspruch 2, wobei die ersten Sensordaten eine Wellenform einschließen, die Änderungen der Herzfrequenz im Verlauf der Zeit angibt;
wobei der erste Spitzenwert eine jüngste Spitze einschließt, die in der Wellenform angegeben ist, und
wobei der zweite Spitzenwert zumindest einen Spitzenwert einschließt, der vor dem Erfassen des ersten Spitzenwerts erfasst wurde, wie in der Wellenform angegeben.

4. Verfahren nach Anspruch 1, wobei das Schätzen der Übungshaltung ferner Folgendes umfasst:
Korrigieren der Haltung der ersten elektronischen Vorrichtung, die durch die zweiten Sensordaten angezeigt wird, basierend auf einer Kalibrierung gegen eine Oberfläche der Erde; und
wobei die Übungshaltung basierend auf einer Änderung des Werts der Haltung der ersten externen elektronischen Vorrichtung geschätzt wird, die durch eine Korrektur verursacht wird, die auf einer Kalibrierung gegen die Oberfläche der Erde basiert.

5. Verfahren nach Anspruch 4, wobei das Korrigieren der Haltung der ersten externen elektronischen Vorrichtung ferner Folgendes umfasst: Konfigurieren von Roll- und Nickwerten, die durch die zweiten Sensordaten angegeben werden, damit sie parallel zu der Oberfläche der Erde und senkrecht zur Schwerkraft sind.

6. Verfahren nach Anspruch 1, wobei das Schätzen der Übungshaltung Folgendes umfasst:
für einen Zeitraum nach dem bestimmten Übungsstartzeitpunkt Erkennen einer Änderung der Haltung der ersten elektronischen Vorrichtung, einschließlich einer Änderung des Rollens und/oder des Nickens, und eines Grads der Änderung, wie in den zweiten Sensordaten angegeben; und
Schätzen einer geänderten Übungshaltung aus einer anfänglichen Haltung, die während des Übungsstartzeitpunkts angezeigt wird, basierend auf der erkannten Änderung der Haltung.

7. Verfahren nach Anspruch 6, wobei die anfängliche Haltung zumindest eines von Folgenden einschließt:
aufrecht senkrecht zu einer Oberfläche der Erde stehen, flach parallel zu der Oberfläche der Erde liegen und mit dem Gesicht nach unten parallel zu der Oberfläche der Erde liegen.

8. Verfahren nach Anspruch 1, wobei das Erkennen des Musters der Abstandsänderung zwischen der Vielzahl von externen elektronischen Vorrichtungen ferner Folgendes einschließt:
Bestimmen, ob das Muster angibt, dass der Abstand zwischen der Vielzahl von externen elektronischen Geräten über den Zeitraum nach dem bestimmten Übungsstartzeitpunkt ansteigt und dann sinkt, oder ob das Muster angibt, dass der Abstand zwischen der Vielzahl von externen elektronischen Vorrichtungen über den Zeitraum nach dem bestimmten Übungsstartzeitpunkt sinkt und dann steigt.

9. Verfahren nach Anspruch 1, wobei die erzeugte Übungsinformation ferner eines von Folgendem einschließt:
eine Benutzerhaltung für eine Übung basierend auf einer Neigung, die durch die geschätzte Übungshaltung angegeben wird; und
eine Anzahl von Wiederholungen für eine Übung, die der Benutzer durchführen soll, basierend auf dem geschätzten Muster der Abstandsänderung zwischen der Vielzahl von externen elektronischen Vorrichtungen.

10. Verfahren nach Anspruch 1, wobei das Verfahren zumindest ferner eines von Folgendem umfasst:
Schätzen einer relativen Position zwischen jeder der Vielzahl von externen elektronischen Vorrichtungen gemäß einer Höhendifferenz zwischen jeder der Vielzahl von externen elektronischen Vorrichtungen, basierend auf Sensordaten, die jeweilige Höhen jeder der Vielzahl von externen elektronischen Vorrichtungen relativ zu einer Oberfläche der Erde angeben, und
Schätzen eines zweiten Musters der Änderung basierend auf vierten Sensordaten, die von jeder der Vielzahl von externen elektronischen Vorrichtungen erfasst werden, die jeweils Bewegungsrichtungen jeder der Vielzahl von externen elektronischen Vorrichtungen angeben.

11. Verfahren nach Anspruch 10, wobei die Übungsinformationen für einen Zielbereich erzeugt werden, der eine Region des Körpers eines Benutzers angibt, auf die durch die Übung abgezielt wird, basierend auf einem oder mehreren von Folgendem:
die geschätzte Übungshaltung, das geschätzte Muster der Abstandsänderung zwischen der Vielzahl von externen elektronischen Vorrichtungen, die geschätzte relative Position zwischen den externen elektronischen Vorrichtungen gemäß der Höhendifferenz und das geschätzte zweite Muster der Änderung, das die Bewegungsrichtung jeder der Vielzahl von externen elektronischen Vorrichtungen angibt.

12. Elektronische Vorrichtung (401), umfassend:
eine Kommunikationsschaltung (190), die konfiguriert ist, mit einer Vielzahl von externen elektronischen Vorrichtungen (402, 403) zu kommunizieren, die an einem Körper eines Benutzers tragbar sind und erste Sensordaten, zweite Sensordaten und dritte Sensordaten von der Vielzahl externer elektronischer Vorrichtungen empfangen;
zumindest einen Prozessor (120), der zu Folgendem konfiguriert ist:
Bestimmen eines Übungsstartzeitpunkts basierend auf den ersten Sensordaten, einschließlich einer Herzfrequenz des Benutzers,
Schätzen einer Übungshaltung des Benutzers basierend auf den zweiten Sensordaten, die eine Haltung einer ersten externen elektronischen Vorrichtung aus der Vielzahl von externen elektronischen Vorrichtungen angeben,
Schätzen eines Musters der Abstandsänderung zwischen jeder der Vielzahl von externen elektronischen Vorrichtungen, angegeben durch Änderungen einer relativen Position zwischen jeder der Vielzahl von externen elektronischen Vorrichtungen über einen Zeitraum nach dem bestimmten Übungsstartzeitpunkt und basierend auf den dritten Sensordaten, die die relative Position zwischen der Vielzahl von externen elektronischen Vorrichtungen angeben, und
Erzeugen von Übungsinformationen basierend auf der geschätzten Übungshaltung und des geschätzten Musters der Abstandsänderung zwischen der Vielzahl von externen elektronischen Vorrichtungen.

13. Elektronische Vorrichtung nach Anspruch 12, wobei das Bestimmen des Übungsstartzeitpunkts Folgendes umfasst:
Erkennen eines ersten Spitzenwerts und eines zweiten Spitzenwerts einer Herzfrequenz basierend auf den ersten Sensordaten;
Vergleichen eines Verhältnisses zwischen dem ersten Spitzenwert und dem zweiten Spitzenwert mit einem vorbestimmten Schwellenwert; und
Bestimmen eines Zeitpunkts, der dem ersten Spitzenwert entspricht, als einen Übungsstartzeitpunkt basierend auf einem Ergebnis des Vergleichens.

14. Elektronische Vorrichtung nach Anspruch 12, wobei das Schätzen der Übungshaltung ferner Folgendes umfasst:
Korrigieren der Haltung einer ersten externen elektronischen Vorrichtung aus der Vielzahl von externen elektronischen Vorrichtungen, wie durch die zweiten Sensordaten basierend auf einer Kalibrierung gegen eine Oberfläche der Erde angegeben; und
wobei die Übungshaltung basierend auf einer Änderung des Werts der Haltung der ersten externen elektronischen Vorrichtung geschätzt wird, die durch eine Korrektur verursacht wird, die auf einer Kalibrierung gegen die Oberfläche der Erde basiert.

15. Tragbare Vorrichtung (1301), die mit zumindest einer externen elektronischen Vorrichtung (1302) zusammenwirkt, die tragbare Vorrichtung umfassend:
eine Vielzahl von Sensoren; und
ein Ausgabemodul;
zumindest einen Prozessor (120), der zu Folgendem konfiguriert ist:
Bestimmen eines Übungsstartzeitpunkts eines Benutzers, der die tragbare Vorrichtung trägt, basierend auf ersten Sensordaten, die über die Vielzahl von Sensoren erfasst werden, einschließlich einer Herzfrequenz des Benutzers,
Schätzen einer Übungshaltung des Benutzers basierend auf zweiten Sensordaten, die eine Haltung der tragbaren Vorrichtung angeben, wie durch die Vielzahl von Sensoren erkannt,
Schätzen eines Musters der Abstandsänderung mit der externen elektronischen Vorrichtung basierend auf dem Übungsstartzeitpunkt und auf dritten Sensordaten, die von der Vielzahl von Sensoren erkannt werden, die den Abstand mit der externen elektronischen Vorrichtung über zumindest einen Zeitraum nach dem Übungsstartzeitpunkt überwachen,
Erzeugen von Übungsinformationen basierend zumindest auf der geschätzten Übungshaltung und des geschätzten Musters der Abstandsänderung mit der externen elektronischen Vorrichtung, und
Ausgeben der erzeugten Übungsinformation über das Ausgabemodul.

## Revendications

1. Procédé de fonctionnement d'un dispositif électronique (401) interagissant avec une pluralité de dispositifs électroniques externes (402, 403) pouvant être portés sur le corps d'un utilisateur, le procédé comprenant :
la réception, par l'intermédiaire d'un circuit de communication (190) provenant de la pluralité de dispositifs électroniques externes, des premières données de capteur comprenant une fréquence cardiaque de l'utilisateur, des deuxièmes données de capteur indiquant une posture d'un premier dispositif électronique externe parmi la pluralité de dispositifs électroniques externes, et des troisièmes données de capteur indiquant une position relative entre la pluralité de dispositifs électroniques externes ;
la détermination (510), par l'intermédiaire d'au moins un processeur (120), d'un instant de début d'exercice sur la base des premières données de capteur ;
l'estimation (520) d'une posture d'exercice de l'utilisateur sur la base des deuxièmes données de capteur ;
l'estimation (530) d'un modèle de changement de distance entre chacun de la pluralité de dispositifs électroniques externes, indiqué par des changements de la position relative entre chacun de la pluralité de dispositifs électroniques externes au cours d'une période de temps après l'instant de début d'exercice déterminé, et sur la base des troisièmes données de capteur ; et
la génération (540) d'informations d'exercice sur la base de la posture d'exercice estimée et du modèle estimé de changement de distance entre la pluralité de dispositifs électroniques externes.

2. Procédé de la revendication 1, ladite détermination de l'instant de début d'exercice comprenant :
la détection d'une première valeur de crête et d'une seconde valeur de crête d'une fréquence cardiaque sur la base des premières données de capteur ;
la comparaison d'un rapport entre la première valeur de crête et la seconde valeur de crête à un seuil prédéfini ; et
la détermination d'un instant correspondant à la première valeur de crête en tant qu'instant de début d'exercice, sur la base d'un résultat de la comparaison.

3. Procédé de la revendication 2, lesdites premières données de capteur comprenant une forme d'onde indiquant des changements de la fréquence cardiaque au cours du temps ;
ladite première valeur de crête comprenant la crête la plus récente indiquée dans la forme d'onde, et
ladite seconde valeur de crête comprenant au moins une valeur de crête acquise avant l'acquisition de la première valeur de crête, comme indiquée dans la forme d'onde.

4. Procédé de la revendication 1, ladite estimation de la posture d'exercice comprenant en outre :
la correction de la posture du premier dispositif électronique indiquée par les deuxièmes données de capteur sur la base de l'étalonnage par rapport à une surface de la terre ; et
ladite posture d'exercice étant estimée sur la base d'un changement de valeur de la posture du premier dispositif électronique externe engendré par une correction sur la base d'un étalonnage par rapport à la surface de la terre.

5. Procédé de la revendication 4, ladite correction de la posture du premier dispositif électronique externe comprenant en outre : la configuration de valeurs de roulis et de tangage indiquées par les deuxièmes données de capteur pour être parallèles à la surface de la terre et perpendiculaires à la gravité.

6. Procédé de la revendication 1, ladite estimation de la posture d'exercice comprenant :
pendant une période de temps après l'instant de début d'exercice déterminé, la détection d'un changement de la posture du premier dispositif électronique, comprenant un changement de roulis et/ou de tangage, et un degré de changement, comme indiqué dans les deuxièmes données de capteur ; et
l'estimation d'une posture d'exercice changée par rapport à une posture initiale indiquée durant l'instant de début d'exercice, sur la base du changement détecté dans la posture.

7. Procédé de la revendication 6, ladite posture initiale comprenant au moins un parmi :
debout perpendiculaire à une surface de la terre, couchée à plat parallèle à la surface de la terre, et couchée face vers le bas parallèle à la surface de la terre.

8. Procédé de la revendication 1, ladite détection du modèle de changement de distance entre la pluralité de dispositifs électroniques externes comprenant en outre :
la détermination pour savoir si le modèle indique que la distance entre la pluralité de dispositifs électroniques externes augmente et ensuite diminue au cours de la période de temps après l'instant de début d'exercice déterminé, ou si le modèle indique que la distance entre la pluralité de dispositifs électroniques externes diminue et ensuite augmente au cours de la période de temps après l'instant de début d'exercice déterminé.

9. Procédé de la revendication 1, lesdites informations d'exercice générées comprenant en outre au moins un parmi :
une posture d'utilisateur pour un exercice sur la base d'une inclinaison indiquée par la posture d'exercice estimée ; et
un nombre de répétitions pour un exercice à réaliser l'utilisateur, sur la base du modèle estimé de changement de distance entre la pluralité de dispositifs électroniques externes.

10. Procédé de la revendication 1, comprenant en outre au moins un parmi :
l'estimation d'une position relative entre chacun de la pluralité de dispositifs électroniques externes selon une différence de hauteur entre chacun de la pluralité de dispositifs électroniques externes, sur la base de données de capteur indiquant des hauteurs respectives de chacun de la pluralité de dispositifs électroniques externes par rapport à une surface de la terre, et
l'estimation d'un second modèle de changement sur la base de quatrièmes données de capteur acquises à partir de chacun de la pluralité de dispositifs électroniques externes, indiquant respectivement les directions de mouvement de chacun de la pluralité de dispositifs électroniques externes.

11. Procédé de la revendication 10, lesdites informations d'exercice étant générées pour une zone cible, indiquant une zone du corps d'un utilisateur ciblée par l'exercice, sur la base d'un ou de plusieurs parmi :
la posture d'exercice estimée, le modèle estimé de changement de la distance entre la pluralité de dispositifs électroniques externes, la position relative estimée entre les dispositifs électroniques externes selon la différence de hauteur, et le second modèle estimé de changement indiquant la direction de mouvement de chacun de la pluralité de dispositifs électroniques externes.

12. Dispositif électronique (401) comprenant :
un circuit de communication (190) configuré pour communiquer avec une pluralité de dispositifs électroniques externes (402, 403) pouvant être portés sur le corps d'un utilisateur et recevoir des premières données de capteur, des deuxièmes données de capteur et des troisièmes données de capteur en provenance de la pluralité de dispositifs électroniques externes ;
au moins un processeur (120) configuré pour :
déterminer un instant de début d'exercice sur la base des premières données de capteur comprenant une fréquence cardiaque de l'utilisateur,
estimer une posture d'exercice de l'utilisateur sur la base des deuxièmes données de capteur indiquant une posture d'un premier dispositif électronique externe parmi la pluralité de dispositifs électroniques externes,
estimer un modèle de changement de distance entre chacun de la pluralité de dispositifs électroniques externes, indiqué par des changements d'une position relative entre chacun de la pluralité de dispositifs électroniques externes sur une période de temps après l'instant de début d'exercice déterminé, et sur la base des troisièmes données de capteur indiquant la position relative entre la pluralité de dispositifs électroniques externes, et
générer des informations d'exercice sur la base de la posture d'exercice estimée et du modèle estimé de changement de distance entre la pluralité de dispositifs électroniques externes.

13. Dispositif électronique de la revendication 12, ladite détermination de l'instant de début d'exercice comprenant :
la détection d'une première valeur de crête et d'une seconde valeur de crête d'une fréquence cardiaque sur la base des premières données de capteur ;
la comparaison d'un rapport entre la première valeur de crête et la seconde valeur de crête à un seuil prédéfini ; et
la détermination d'un instant correspondant à la première valeur de crête en tant qu'instant de début d'exercice, sur la base d'un résultat de la comparaison.

14. Dispositif électronique de la revendication 12, ladite estimation de la posture d'exercice comprenant en outre :
la correction de la posture d'un premier dispositif électronique externe parmi la pluralité de dispositifs électroniques externes, comme indiqué par les deuxièmes données de capteur sur la base de l'étalonnage par rapport à une surface de la terre ; et
ladite posture d'exercice étant estimée sur la base d'un changement de valeur de la posture du premier dispositif électronique externe engendré par une correction sur la base d'un étalonnage par rapport à la surface de la terre.

15. Dispositif pouvant être porté (1301) interagissant avec au moins un dispositif électronique externe (1302), le dispositif pouvant être porté comprenant :
une pluralité de capteurs ; et
un module de sortie ;
au moins un processeur (120) configuré pour :
déterminer un instant de début d'exercice d'un utilisateur portant le dispositif pouvant être porté sur la base de premières données de capteur détectées par l'intermédiaire de la pluralité de capteurs, comprenant une fréquence cardiaque de l'utilisateur,
estimer une posture d'exercice de l'utilisateur sur la base de deuxièmes données de capteur indiquant une posture du dispositif pouvant être porté telle que détectée par la pluralité de capteurs,
estimer un modèle de changement de distance avec le dispositif électronique externe sur la base de l'instant de début d'exercice, et des troisièmes données de capteur détectées par la pluralité de capteurs surveillant la distance avec le dispositif électronique externe sur au cours d'au moins une période de temps après l'instant de début d'exercice,
générer des informations d'exercice sur la base au moins de la posture d'exercice estimée et du modèle estimé de changement de la distance avec le dispositif électronique externe, et
délivrer en sortie les informations d'exercice générées par l'intermédiaire du module de sortie.
